# EUROPEAN PATENT SPECIFICATION

(11) **EP 4 199 731 B1**
(45) Date of publication and mention of the grant of the patent: **09.12.2026**
(21) Application number: 21858813.5
(22) Date of filing: 05.08.2021
(51) Int. Cl.: A23D 9/00, A23K 10/00, A23K 20/142, A23K 50/10, A23L 5/00, G06Q 50/02, G16H 20/60

(54) **SYSTEMS AND METHODS FOR FORMULATING RUMINANT DIETS**
SYSTEME UND VERFAHREN ZUR FORMULIERUNG VON ERNÄHRUNG FÜR WIEDERKÄUER
SYSTÈMES ET PROCÉDÉS DE FORMULATION DE RÉGIMES ALIMENTAIRES DE RUMINANT

(30) Priority: 19.08.2020 US 202063067721 P
(43) Date of publication of application: 28.06.2023
(73) Proprietor: Boveta Nutrition LLC, Atlanta, GA 30305 (US)
(72) Inventor: KERLEY, Monty S., Atlanta, Georgia 30305 (US)
(74) Representative: J A Kemp LLP
(86) International application number: PCT/US2021/044793
(87) International publication number: WO 2022/039942

(56) References cited:
- WO-A1-2020/167986
- US-A1- 2004 170 669
- US-A1- 2008 026 129
- US-A1- 2014 274 886
- US-A1- 2015 164 110
- US-A1- 2016 324 188
- US-A1- 2020 260 759
- DOEPEL L ET AL: "Milk Protein Synthesis as a Function of Amino Acid Supply", JOURNAL OF DAIRY SCIENCE, AMERICAN DAIRY SCIENCE ASSOCIATION, US, vol. 87, no. 5, 1 May 2004 (2004-05-01), pages 1279 - 1297, XP026974606, ISSN: 0022-0302, [retrieved on 20040501]
- N. SWANEPOEL ET AL: "Amino acid needs of lactating dairy cows: Impact of feeding lysine in a ruminally protected form on productivity of lactating dairy cows", ANIMAL FEED SCIENCE AND TECHNOLOGY, vol. 157, no. 1-2, 1 April 2010 (2010-04-01), AMSTERDAM, NL, pages 79 - 94, XP055690203, ISSN: 0377-8401, DOI: 10.1016/j.anifeedsci.2010.02.008
- ROBINSON P H ET AL: "Effects of feeding a ruminally protected lysine product, with or without isoleucine, valine and histidine, to lactating dairy cows on their productive performance and plasma amino acid profiles", ANIMAL FEED SCIENCE AND TECHNOLOGY, ELSEVIER, AMSTERDAM, NL, vol. 161, no. 3-4, 3 November 2010 (2010-11-03), pages 75 - 84, XP027446292, ISSN: 0377-8401, [retrieved on 20101027], DOI: 10.1016/J.ANIFEEDSCI.2010.07.017
- RIUS ET AL.: "Interactions of energy and predicted metabolizable protein in determining nitrogen efficiency in the lactating dairy cow", JOURNAL OF DAIRY SCIENCE, vol. 93, 2010, pages 2034 - 2043, XP027015861, [retrieved on 20210927], DOI: https://doi.org/10.3168/jds.2008-1777

## Description

### FIELD

The present disclosure relates generally to ruminant diets, and more particularly, to systems and methods for formulating an adjusted diet, a dietary supplement, or both, for ruminants, as well as systems and methods for administering the adjusted diet, dietary supplement, or both to a ruminant. The disclosure also relates to non-transitory computer-readable media storing computer-executable instructions relating to the methods

The invention pertains to the subject-matter of the claims.

### BACKGROUND

For efficient production of human edible products, ruminants should be fed nutritionally adequate feedstuffs prepared and delivered in formulated diets. All dietary components, such as energy, amino acids (AA), vitamins, and minerals are important when formulating the ruminant diet. However, more attention should be given to the dietary energy, protein and AA, as these components account for a substantial proportion of dietary amount (%) and cost. Deficiencies or imbalances of essential AA and energy can lead to reductions in animal performance and increases in the excretion and loss of valuable nutrients in manure and exacerbate emissions of gaseous nitrogen and methane, which are implicated as greenhouse gases (GHG) contributing to climate change. Furthermore, these gases may be respiratory irritants and are thus detrimental to animal and human health. Therefore, it is especially important to formulate and deliver diets to meet ruminant energy and AA requirements while minimizing overfeeding and excretion of excess nutrients.

Embodiments of the disclosed subject matter may address one or more of the above-noted problems and disadvantages, among other things.

US 2014/0274886 A1 describes a method wherein an amount of rumen undegraded protein is determined by analysis of feed components in a ruminant feed, and is then used to modify the feed formulation to control the level of rumen undegrated protein exiting the rumen of the ruminant. The rumen undegraded neutral detergent fibre content of selected feed components may also be determined based on an analysis of the feed components. The rumen undegraded protein content may be determined by comparing analysis results with a calibration curve of previously analyzed feed components.

### SUMMARY

This disclosure concerns embodiments of systems and methods for determining and preparing ruminant diets more accurately for provision of amino acids (AA) relative to ruminant intake of usable energy. Ruminant animals fed more precisely formulated diets demonstrate improved feed efficiency, improved output of usable products, or both. A further benefit is the reduction in manure and greenhouse gases produced per unit of usable product produced. And still further, the nutritional value of edible products is improved by provision of a more balanced and nourishing diet to the growing or lactating ruminant.

The method, system and one or more non-transitory computer-readable media of the invention are as defined in the claims.

The method may be suitable for preparing an adjusted diet, a dietary supplement, or both for a ruminant. The method includes determining an amount of urea, a protein source, peptides, rumen-protected peptides (RPP), rumen-protected amino acids (RPAA), or any combination thereof, to add to an unaltered diet of the ruminant to provide an amino acid-balanced diet that meets an amino acid requirement of the ruminant based at least in part upon usable energy consumed by the ruminant and the energy requirement of the ruminant, wherein the usable energy is effective energy (EE) or metabolizable energy (ME) and the energy requirement is an effective energy requirement (EERQ) or metabolizable energy requirement (MERQ), respectively. This determination is based at least in part on a comparison between predicted intestinal supply of absorbable dietary amino acids and microbial amino acids and the amino acid requirements of the ruminant. At least part of the method is performed by one or more computing devices. The method may further comprise preparing an adjusted diet, a dietary supplement, or a combination thereof, comprising the amount of urea, protein source, peptides, RPP, RPAA, or combination thereof. The method may further include determining a quantity of usable energy provided by an amount of the unaltered diet consumed by the ruminant. In some embodiments, the quantity of usable energy is determined from the protein, starch, and fiber content of the unaltered diet. In certain embodiments, the ruminant is a bovine, ovine, or caprine.

In any of the above embodiments, the method may further include determining the EERQ or MERQ of the ruminant. EERQ is based on the requirements for maintenance heat energy, protein accretion energy, lipid accretion energy, and methane gas energy. MERQ is based on the requirements for maintenance heat energy, protein accretion energy, lipid accretion energy, methane gas energy, and lactation energy. In any of the above embodiments, the method may further include determining the amino acid requirement of the ruminant based at least in part on the EERQ or MERQ for the ruminant and the quantity of EE or ME, respectively, provided by the amount of the unaltered diet consumed by the ruminant.

In any of the above embodiments, the method may further include determining ruminal microbial efficiency. In some embodiments, determining ruminal microbial efficiency is based at least in part on dilution rate, which is the percentage of the rumen volume that passes from the rumen per unit of time. In certain embodiments, predicting dietary amino acid and microbial amino acid flow to the small intestine is based at least in part on ruminal microbial efficiency, the quantity of usable energy provided by the amount of the unaltered diet consumed by the ruminant, and the protein, starch, and fiber content of the unaltered diet.

In any of the above embodiments, the method may further include determining a rumen microbial peptide-nitrogen or amino acid-nitrogen requirement and a rumen microbial ammonia-nitrogen requirement. In some embodiments, the method also includes comparing the peptide- or amino acid-nitrogen nitrogen supplied by the unaltered diet to the microbial peptide- or amino acid-nitrogen nitrogen requirement to provide a peptide- or amino acid-nitrogen nitrogen comparison. The ammonia-nitrogen supplied by the unaltered diet also can be compared to the microbial ammonia-nitrogen requirement to provide an ammonia-nitrogen comparison. In such embodiments, determining the amount of urea, protein source, peptides, RPP, RPAA, or any combination thereof, further may be based at least in part on the peptide nitrogen comparison and the ammonia-nitrogen comparison.

Dietary supplements prepared by the disclosed methods may include urea, a protein source, peptides, RPP, RPAA, or any combination thereof. Relative to the ruminant's unaltered diet, an adjusted diet prepared by the disclosed methods may include an additional protein source, a different protein source, an adjusted amount of a protein source, a reduced amount of roughage, or a combination thereof, relative to the unaltered diet.

A ruminant may be administered (i) an amount of an adjusted diet prepared by the disclosed methods, or (ii) an amount of an unaltered diet and an amount of a dietary supplement prepared by the disclosed methods, or (iii) an amount of an adjusted diet and an amount of a dietary supplement prepared by the disclosed methods. In some embodiments, (i) the amount of the adjusted diet is effective to mitigate an amino acid deficiency of the unaltered diet, or (ii) the amount of the dietary supplement is effective to mitigate an amino acid deficiency of the unaltered diet, or (iii) the amount of the adjusted diet and the amount of the dietary supplement in combination are effective to mitigate an amino acid deficiency of the unaltered diet.

In any of the above embodiments, the adjusted diet, the dietary supplement, or the adjusted diet and the dietary supplement may be administered periodically as desired and as effective, typically at least once daily. The dietary supplement may be administered to the ruminant by combining the dietary supplement with the ruminant's unaltered diet or administering independently of the unaltered diet a dietary supplement to augment an unaltered diet fed to the ruminant. The adjusted diet may be administered to the ruminant by replacing the ruminant's unaltered diet with the adjusted diet or by combining the adjusted diet with the ruminant's unaltered diet.

In any of the foregoing embodiments, administering the adjusted diet, the unaltered diet and the amount of the dietary supplement, or the adjusted diet and the amount of the dietary supplement to the ruminant, provides substantial benefits compared to a ruminant that did not receive the adjusted diet, the dietary supplement, or the adjusted diet and the dietary supplement. For example, in the case of a growing ruminant fed for meat production or a non-lactating female ruminant, such administrations may increase the ruminant's rate of gain, decrease the ruminant's dry matter intake, increase the ruminant's feed efficiency, decrease the ruminant's cost of gain, decrease the ruminant's methane gas generation, reduce the ruminant's age of reproductive development, improve the ruminant's reproductive success, increase the ruminant's yield of edible carcass, increase the ruminant's carcass yield or quality grade, or any combination thereof. In some embodiments, the ruminant is a lactating ruminant and administering the adjusted diet, the unaltered diet and the amount of the dietary supplement, or the adjusted diet and the amount of the dietary supplement to the ruminant increases the ruminant's milk yield, increases the ruminant's milk efficiency, increases the ruminant's milk components, increases income over feed cost, reduces the ruminant's milk somatic cell count, or any combination thereof compared to a dairy ruminant that did not receive the adjusted diet, the dietary supplement, or the adjusted diet and the dietary supplement.

At least part of the method is performed by one or more computing devices.

Also provided are one or more non-transitory computer-readable media that can store computer-executable instructions that, when executed by one or more computing devices configure the one or more computing devices to perform at least part of the methods of any of the foregoing embodiments.

Also provided is a system that comprises one or more processors and a computer-readable storage media. The computer-readable storage media stores computer-executable instructions that, when executed by the one or more processors, cause the one or more processors to collectively determine an amount of urea, a protein source, peptides, rumen-protected peptides, rumen-protected amino acids, or any combination thereof, to add to an initial ruminant diet to provide an amino acid-balanced diet that satisfies a ruminant's amino acid requirement. The determined amount is based at least in part on an amino acid comparison between (i) a prediction of dietary amino acid and microbial amino acid flow to the ruminant's small intestine, and (ii) the ruminant's amino acid requirement. The ruminant's amino acid requirement is based at least in part upon usable energy consumed by the ruminant and an energy requirement of the ruminant. The usable energy can be effective energy and the energy requirement can be an effective energy requirement based on the ruminant's requirements for maintenance heat energy, protein accretion energy, lipid accretion energy, and methane gas energy. Alternatively, the usable energy can be metabolizable energy and the energy requirement can be a metabolizable energy requirement based on the ruminant's requirements for maintenance heat energy, protein accretion energy, lipid accretion energy, methane gas energy, and lactation energy.

The foregoing and other objects, features, and advantages of the invention will become more apparent from the following detailed description, which proceeds with reference to the accompanying figures.

### BRIEF DESCRIPTION OF THE DRAWINGS

Embodiments will hereinafter be described with reference to the accompanying drawings, which have not necessarily been drawn to scale. Where applicable, some elements may be simplified or otherwise not illustrated in order to assist in the illustration and description of underlying features. Throughout the figures, like reference numerals denote like elements.
FIG. 1 is a flowchart illustrating one embodiment of a method for preparing an adjusted diet and/or dietary supplement for a ruminant.
FIG. 2 is a flowchart illustrating another embodiment of a method for preparing an adjusted diet and/or dietary supplement for a ruminant.
FIG. 3 is a flowchart illustrating yet another embodiment of a method for preparing an adjusted diet and/or dietary supplement for a ruminant.
FIG. 4 is a simplified schematic diagram illustrating aspects of a computing device for determining an adjusted diet and/or dietary supplement for a ruminant, according to one or more embodiments of the disclosed subject matter.
FIG. 5 is a simplified schematic diagram illustrating aspects of a computerized system for formulating a diet for a ruminant, according to one or more embodiments of the disclosed subject matter.
FIG. 6 is a process flow diagram illustrating aspects of a computer-implemented method for formulating a diet for ruminant, according to one or more embodiments of the disclosed subject matter.
FIG. 7 depicts a generalized example of a suitable computing environment in which the described innovations may be implemented.

### DETAILED DESCRIPTION

Amino acids (AA) are the building blocks for protein synthesis but there exists uncertainty in how to formulate ruminant diets to provide the ideal profile of absorbed AA for animal maintenance, growth, pregnancy and milk production. Furthermore, the ideal profile of AA differs based upon genetics, animal life-cycle stage and physiological state (e.g., high vs. low milk secretion). Provision of a more balanced profile of absorbable AA allows meeting AA requirements with less dietary protein or using more economical proteins in the diet or both. By selective use of protein supplements and ruminally-protected AA such as Lys and Met, AA requirements can be satisfied with lower concentrations of dietary protein.

Balancing diets for AA can reduce the amount of total protein fed and more specifically the amount of protein that is resistant to fermentation in the rumen, which is defined as rumen undegraded protein (RUP). However, the quality (AA profile) of RUP becomes paramount for optimizing absorbable AA supply when lesser amounts of RUP are fed. Furthermore, because microbial protein provides about 50% of the absorbed AA requirements of ruminants, it is important to provide adequate amounts of ruminally degraded protein (RDP) to meet the nitrogen requirement of rumen microorganisms. The RDP advantageously is comprised of ammonia and degradable amino acids and peptides to maximize efficiency of microbial protein production.

Existing diet formulation methods have inaccuracies related to understanding and modeling the biology of ruminant growth and lactation. These inaccuracies include but are not limited to, inaccurate energy requirement calculations for growth, inaccurate modeling of microbial growth in the rumen that results in inaccurate prediction of the microbial requirements for ammonia-nitrogen and peptide and amino acid nitrogen required to maximize microbial growth, and/or inaccurate prediction of post ruminal flow of dietary amino acids and rumen synthesized microbial amino acids attributed to inaccurate prediction of dietary protein, fiber, and starch fermentation in the rumen.

To illustrate the problem with current formulation practices, Santos et al (J of Dairy Science 1998, 81(12):3182-3213) summarized published research for lactating ruminants and found that in 127 comparisons from 88 lactation trials, milk yield was higher for diets containing greater amounts of RUP in only 17% of the comparisons. Furthermore, milk protein was increased in only 5% of the comparisons while being decreased in 22%, of the comparisons. The problem in many of the experiments was the failure to recognize or respect key factors affecting performance, such as the quality of the RUP and specifically the provision of digestible AA in ideal amounts and proportion relative to energy content of the diet. In some studies, it was possible that RUP was not limiting animal performance, or worse, when RUP was increased, it came at the expense of RDP, causing a deficiency of RDP. Insufficient RDP can decrease microbial activity in the rumen, leading to worse digestion of consumed feeds, particularly the energy components of the diet (starch, fiber). Furthermore, reduced microbial activity or poorer efficiency of microbial protein synthesis reduces the synthesis and flow of microbial AA to the intestines, which diminishes the amount and quality of AA available to support the productive function of the animal.

The effective energy requirement (EERQ) is a more accurate reflection of the ruminant's energy requirements than the commonly used net energy requirement. Net energy requirement analysis assumes that energy requirements for protein and lipid accretion are the same and typically over-predicts the ruminant's energy requirement by 20% or more. Whereas EERQ more accurately describes energy requirement compared with the Net Energy system, a limitation of EERQ is that it fails to integrate protein feeding strategies as it does not address how to formulate diets to optimize provision of AA from rumen microbial protein synthesis and RUP in the context of Effective Energy (EE) intake.

This disclosure concerns embodiments of a method for determining and preparing an adjusted diet and/or dietary supplement for ruminants, and for administering the adjusted diet and/or dietary supplement to a ruminant. In some embodiments, the disclosed method and adjusted diet/dietary supplement provide the ruminant with an amino acid-balanced diet, thereby supporting ruminant growth or milk production and/or improving product value, relative to a ruminant that does not receive the adjusted diet and/or dietary supplement. In some embodiments, the determining and/or preparing can be performed by a computer system

The invention pertains to the subject-matter of the claims.

### I. Definitions and Abbreviations

The following explanations of terms and abbreviations are provided to better describe the present disclosure and to guide those of ordinary skill in the art in the practice of the present disclosure. As used herein, "comprising" means "including" and the singular forms "a" or "an" or "the" include plural references unless the context clearly dictates otherwise. The term "or" refers to a single element of stated alternative elements or a combination of two or more elements, unless the context clearly indicates otherwise.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting. Other features of the disclosure are apparent from the following detailed description and the claims.

The disclosure of numerical ranges should be understood as referring to each discrete point within the range, inclusive of endpoints, unless otherwise noted. Unless otherwise indicated, all numbers expressing quantities of components, percentages, temperatures, times, and so forth, as used in the specification or claims are to be understood as being modified by the term "about." Accordingly, unless otherwise implicitly or explicitly indicated, or unless the context is properly understood by a person of ordinary skill in the art to have a more definitive construction, the numerical parameters set forth are approximations that may depend on the desired properties sought and/or limits of detection under standard test conditions/methods as known to those of ordinary skill in the art. When directly and explicitly distinguishing embodiments from discussed prior art, the embodiment numbers are not approximates unless the word "about" is recited.

Although there are alternatives for various components, parameters, operating conditions, etc. set forth herein, that does not mean that those alternatives are necessarily equivalent and/or perform equally well. Nor does it mean that the alternatives are listed in a preferred order unless stated otherwise.

Definitions of common terms in chemistry may be found in Richard J. Lewis, Sr. (ed.), Hawley's Condensed Chemical Dictionary, published by John Wiley & Sons, Inc., 2016 (ISBN 978-1-118-13515-0).

In order to facilitate review of the various embodiments of the disclosure, the following explanations of specific terms are provided:
**Adjusted diet:** As used herein, the term "adjusted diet" refers to a diet that has been modified, typically to alter the energy or amino acid content.

**Administering:** Administration by any route to a subject (e.g., a ruminant). As used herein, administration typically but not necessarily refers to oral administration.

**Amino acid balanced diet:** As used herein, the term "amino acid balanced diet" means a diet that includes sufficient amounts of amino acids, particularly essential amino acids, to meet a ruminant's amino acid requirement for maintenance of existing body weight and metabolic functions and sufficient to support additional productive functions such as weight gain and milk secretion.

**Carcass dressing percentage:** (carcass weight/shrunk live weight) × 100
**Cost of gain:** The cost of maintaining an animal (e.g., feed, housing, veterinary care, and the like) for a period of time divided by a weight gain of the animal over the period of time.

Diet: As used herein, the term "diet" refers to anything that may be consumed by an animal. The term "diet" encompasses solid and liquid animal feeds *(e.g.,* a feed ration), water, and feed additive carriers (e.g., molasses).

Dilution rate (DR): The percentage of the rumen volume that passes from the rumen per unit of time. Dilution rate may be determined for insoluble (solid) material, soluble solid materials, and liquids passing from the rumen.

Direct-fed microbial (DFM): Microorganisms, or a product that comprises live microorganisms (e.g., bacteria and/or yeast), also referred to as probiotics, administered as a feed supplement.

Effective energy requirement (EERQ): An estimate of an animal's energy requirement based on energy expenditure for maintenance, protein accretion, lipid accretion, and methane gas evolution.

Essential amino acid: Amino acids that cannot be made by the body or synthesized in adequate quantities. There are ten essential amino acids for ruminants namely methionine, lysine, arginine, histidine, phenylalanine, valine, threonine, tryptophan, leucine, and isoleucine.

Feed efficiency: A measure of an animal's efficiency in converting feed mass into the desired output, e.g., weight gain, milk production. Feed efficiency also may be referred to as feed conversion ratio, feed conversion rate, or feed conversion efficiency.

Feed to gain ratio (feed conversion ratio): A measure of an animal's efficiency in converting feed mass into increased body mass.

Growth promotant: An agent that increases the efficiency of animal production, such as by increasing the rate of weight gain, improved feed efficiency and/or product output. A growth promotant may also increase the quality of a product, such as increase the quality of meat produced. The growth promotant may be a hormone, an antimicrobial, a direct-fed microbial, a beta agonist, a plant extract, or any combination thereof. In some embodiments, the antimicrobial is an ionophore, an antibiotic, an antifungal agent, an antiviral agent, an antiparasitic agent, or any combination thereof.

Hot carcass weight (HCW): The carcass weight minus the weight of the hide, head, feet, and gastrointestinal tract.

Lipid accretion: The process of increasing lipid mass in an animal, e.g., adding lipid mass, such as fat stores, as the animal grows.

Metabolizable energy requirement (MERQ): An estimate of an animal's energy requirement based on energy expenditure for maintenance, protein accretion, lipid accretion, methane gas evolution, and lactation.

**Microbial** amino acids: Amino acids synthesized by ruminal micro-organisms from the process of fermentation and formation of microbial protein from feedstuffs containing carbohydrates and nitrogen.

**Microbial efficiency (MOEFF):** Grams of microbial protein produced per kilogram of dietary organic matter fermented in the rumen.

Milk efficiency: Calculated by dividing milk secretion of a lactating animal by food consumed per unit of time.

Mitigate: As used herein, "mitigate" means to lessen or eliminate.

Plant extract: A substance or an active molecule with desirable properties that is removed from the tissue of a plant, usually by treating it with a solvent, to be used for a particular purpose. In some embodiments, the plant extract is a concentrated hydrophobic liquid containing volatile aromatic compounds.

Protein accretion: The process of increasing protein mass in an animal, e.g., adding protein, such as muscle protein, as the animal grows.

Protein source: Proteins are large biomolecules, or macromolecules, consisting of one or more long chains of amino acid residues. The term protein source encompasses sources of both natural proteins and altered proteins. Altered proteins may be chemically and/or physically treated to affect tertiary structure, fluid solubility and/or characteristics related to fermentation by micro-organisms in the rumen.

Rumen-protected (rumen-bypass) amino acid or peptide: Rumen-protected, or rumen-bypass, amino acids and peptides are formulated to pass through the rumen substantially intact, with subsequent hydrolysis and release of the amino acid in the true stomach or small intestine. Rumen-protected amino acids include, but are not limited to, lipid coated amino acids, lipid-amino acid matrices, pH-sensitive polymer-encapsulated amino acids and protein-protected amino acids.

**Ruminant:** A suborder of mammals, most of which have four-chambered stomachs, including the rumen, reticulum, omasum, and abomasum.

**Shrunk live weight:** The weight of an animal after transportation from a feedlot, dairy operation, or the like to a processing facility.

### II. Preparing Adjusted Diets and/or Dietary Supplements

An adjusted diet and/or dietary supplement is formulated to provide a ruminant with a balanced diet. More specifically, the initial, or unaltered, diet is adjusted to mitigate amino acid deficiencies, thereby providing an amino acid-balanced diet that meets an amino acid requirement based upon usable energy consumed by the ruminant and the energy requirement of the ruminant. For growing ruminants, usable energy is defined as effective energy (EE) whereas for lactating ruminants, usable energy is defined as metabolizable energy (ME). Deficiencies in the amino acids provided by the unaltered diet are mitigated by adjusting the diet and/or administering a dietary supplement to the ruminant.

An adjusted diet is a conventional diet that is altered to provide a different amount of one or more amino acids. The unaltered diet may be adjusted by, for example, utilizing a different protein source, increasing an amount of a protein source, or a combination thereof, to provide the adjusted diet. A dietary supplement includes a sufficient amount of urea, protein, peptides, rumen-protected peptides (RPP), rumen-protected amino acids (RPAA), or any combination thereof to mitigate amino acid deficiencies resulting from the ruminant's unaltered diet and to meet the ruminant's EE or ME requirement. In some embodiments, a combination of an adjusted diet and a dietary supplement provides an amino acid-balanced diet and meets the ruminant's amino acid requirement. The ruminant may be any mammal having a four-chambered stomach. Exemplary ruminants include bovines, ovines, caprines, antelopes, deer, and giraffes. In some embodiments, the ruminant is a bovine such as weaned calf, a non-pregnant female, a castrated male, or a lactating female. In other embodiments, the ruminant is an ovine, such as a lamb, a ewe, a castrated male, or a ram. In still other embodiments, the ruminant is a caprine, such as a kid, a ewe, a castrated male, or a buck.

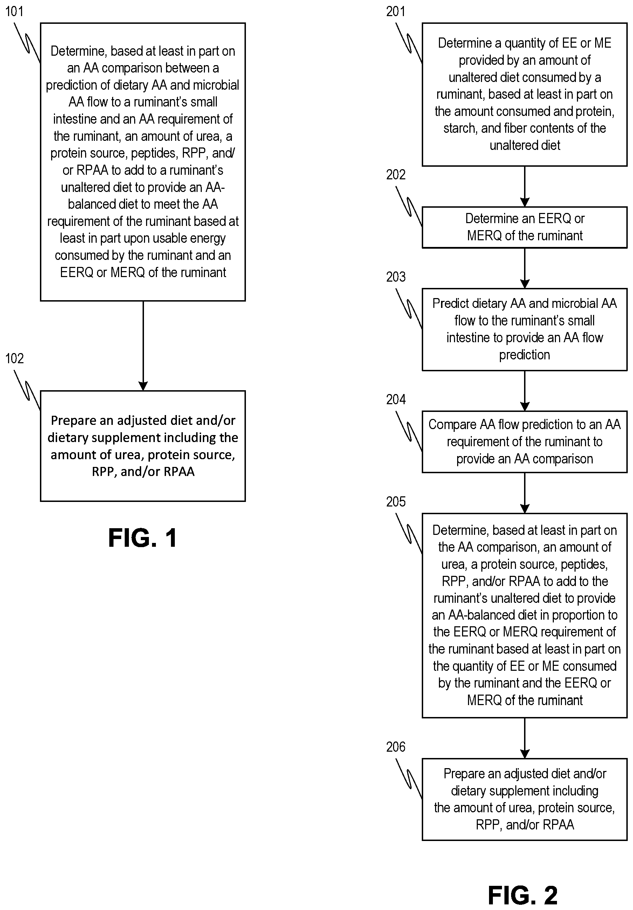

With reference to FIG. 1, the method comprises determining an amount of urea, protein, peptides, RPP, RPAA, or any combination thereof, to add to the ruminant's diet to provide an amino acid (AA) balanced diet that meets an amino acid requirement of the ruminant based upon EE or ME consumed by the ruminant and an EE requirement (EERQ) or ME requirement (MERQ) of the ruminant. This determination is based at least in part on an amino acid comparison between a prediction of dietary amino acid and microbial amino acid flow to a ruminant's small intestine and the amino acid requirement of the ruminant (101). An adjusted diet, a dietary supplement, or a combination thereof, comprising the amount of urea, protein source, peptides, RPP, RPAA, or combination thereof, is prepared for administration to the ruminant (102).

Preparing an adjusted diet may include adding a protein source to the diet, changing a protein source in the diet (replacing a protein source in the unaltered diet with another protein source), adjusting an amount of a protein source present in the diet, reducing an amount of roughage in the diet, or any combination thereof. Preparing a dietary supplement, may include preparing a powder supplement, a granular supplement, a pelleted supplement, a liquid supplement, a dosage form (e.g., a tablet, a capsule), or any combination thereof, wherein the supplement comprises urea, a protein source, peptides, RPP, RPAA, or any combination thereof.

In some embodiments, the method further includes determining a quantity of usable energy (EE or ME) provided by an amount of the unaltered diet consumed by the ruminant. Determining the quantity of usable energy provided by the unaltered diet further comprises determining protein, starch, and fiber content of the unaltered diet.

In certain embodiments, the EE provided by each kilogram of the unaltered diet is based on a ME and protein provided by the unaltered diet.

In any of the foregoing embodiments, the method may further include determining the ruminant's energy requirement. The energy requirement may be an EERQ or MERQ. In some embodiments, EERQ is determined for growing ruminants based on energy required for maintenance, protein accretion energy, lipid accretion energy, and methane gas evolution. In some embodiments, MERQ is determined for lactating ruminants based on energy required for maintenance, protein accretion energy, lipid accretion energy, methane gas evolution, and lactation energy. In any of the foregoing embodiments, the ruminant's amino acid requirement may be based at least in part on the ruminant's EERQ or MERQ and the quantity of EE or ME, respectively provided by the amount of unaltered diet consumed by the ruminant, e.g., the difference between the EERQ or MERQ and the usable energy provided by the unaltered diet. EE consumed is used to first satisfy the ruminant's maintenance energy requirement and remaining energy can be used for growth. The amino acid requirement thus is based at least in part on the mass of protein growth supported by the energy available for growth and the amino acids required for protein tissue accretion, along with amino acids required for maintenance functions. ME consumed is used to first satisfy the ruminant's maintenance energy requirement, and remaining energy can be used for milk production; if sufficient energy is available, remaining energy further can be used for growth. Energy consumption drives growth and/or milk production potential, and amino acids are supplied to support growth and/or milk production potential.

In any of the foregoing embodiments, the method may further include determining ruminal microbial efficiency (MOEFF). In such embodiments, predicting dietary amino acid and microbial amino acid flow to the ruminant's small intestine may be based at least in part on the ruminal microbial efficiency, the quantity of EE or ME provided by the amount of the unaltered diet consumed by the ruminant, and the protein, starch, and fiber content of the unaltered diet. MOEFF is the grams of microbial nitrogen produced per kilogram of dietary organic matter fermented corrected for the contribution of microbial organic matter to ruminal outflow of organic matter, thus referred to as dietary organic matter truly fermented (OMTF). In some embodiments, determining MOEFF is based at least in part on the ruminal dilution rate (DR), in particular based at least in part on the solid dilution rate (SDR). Dilution rate affects MOEFF by affecting microbial growth rate (e.g., by influencing the proportion of ruminal bacteria in the growth phase), microbial maintenance requirements, energy availability (e.g., availability of ATP, ammonia-nitrogen, peptides, and carbohydrates), and/or nitrogen recycling within the rumen. In a steady state environment, microbial growth rate may be equal to the DR. Dilution rate influences ATP availability by influencing the amount of time that a feedstuff is present in the rumen for fermentation. Dilution rate may be influenced by amount of food consumed per meal, ingredients used to form the diet, e.g., fibrous feedstuffs vs. starchy feedstuffs, particle size of feedstuffs mixed into the diet, number of meals consumed per day, and animal-to-animal variation.

In any of the foregoing embodiments, the method may further include determining a rumen microbial peptide-nitrogen or amino acid-nitrogen requirement and/or a rumen microbial ammonia-nitrogen requirement. These requirements are based, at least in part, on MOEFF and the amount of microbial protein synthesized. The ruminal microbes require ammonia nitrogen to ferment fiber, a combination of ammonia nitrogen and peptide nitrogen (2:1 ratio) to ferment starch, and peptide nitrogen to digest protein. The peptide- or amino acid-nitrogen and ammonia-nitrogen supplied by the unaltered diet are also determined and compared to the requirements to provide a peptide- or amino acid-nitrogen comparison and an ammonia-nitrogen comparison. In some embodiments, determining the amount of urea, protein, peptides, RPP, RPAA, or any combination thereof, is further based at least in part on the peptide nitrogen comparison and the ammonia-nitrogen comparison.

In some embodiments, the method includes preparing a dietary supplement to provide the amount of urea, protein source, peptides, RPP, RPAA, or any combination thereof. In one embodiment, the dietary supplement comprises urea. In another embodiment, the dietary supplement comprises a protein source, peptides, RPP, RPAA, or any combination thereof. Exemplary protein sources include, but are not limited to, fishmeal, bloodmeal, soybean meal, canola meal, cottonseed meal, corn gluten feed, dried distillers grains, and combinations thereof.

In another embodiment, the dietary supplement comprises RPAA. In some examples, the RPAA are essential amino acids, namely methionine, lysine, arginine, histidine, phenylalanine, valine, threonine, tryptophan, leucine, isoleucine or any combination thereof.

In one embodiment, with reference to FIG. 2, a method for formulating a dietary supplement is disclosed. Such embodiments may include (201) determining a quantity of EE or ME provided by a diet consumed by a ruminant based at least in part on an amount of the unaltered diet consumed by the ruminant and the protein, starch, and fiber content of the unaltered diet; (202) determining an EE requirement (EERQ) or ME requirement (MERQ) for the ruminant, the EERQ including considering a maintenance heat energy, protein accretion energy, lipid accretion energy, and methane gas energy, and the MERQ including considering a maintenance heat energy, protein accretion energy, lipid accretion energy, and methane gas energy and lactation energy; (203) predicting dietary amino acid and microbial amino acid flow to the ruminant's small intestine to provide an amino acid flow prediction; (204) comparing the amino acid flow prediction to an amino acid requirement of the ruminant to provide an amino acid comparison; (205) determining, based at least in part on the amino acid comparison, an amount of urea, a protein source, peptides, RPP, RPAA, or any combination thereof, to add to the ruminant's diet to provide an amino acid-balanced diet in proportion to the EE or ME requirement of the ruminant; and (206) preparing an adjusted diet, a dietary supplement, or a combination thereof, comprising the amount of urea, protein source, peptides, RPP, RPAA, or combination thereof.

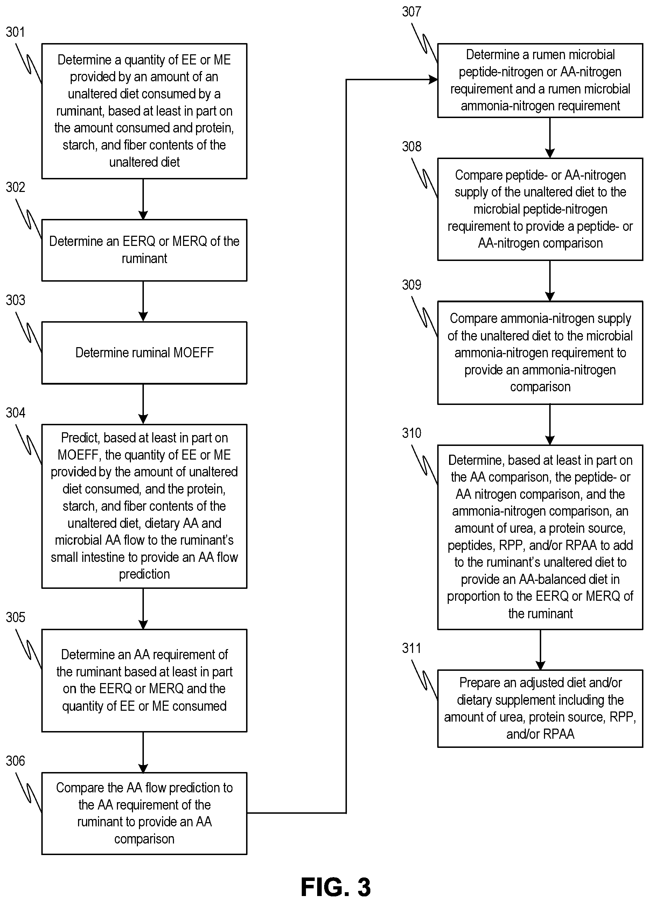

In another embodiment, with reference to FIG. 3, a method for formulating a dietary supplement includes (301) determining a quantity of EE or ME provided by an amount of a diet consumed by the ruminant based at least in part on the amount of the unaltered diet consumed, and the protein, starch, and fiber content of the unaltered diet; (302) determining an EE requirement (EERQ) or ME requirement (MERQ) for the ruminant, the EERQ including considering a maintenance heat energy, protein accretion energy, lipid accretion energy, and methane gas energy and the MERQ including considering a maintenance heat energy, protein accretion energy, lipid accretion energy, and methane gas energy and lactation energy; (303) determining ruminal microbial efficiency; (304) predicting, based at least in part on the ruminal microbial efficiency, the quantity of EE or ME provided by the amount of the unaltered diet consumed, and the protein, starch, and fiber content of the unaltered diet, a dietary amino acid and microbial amino acid flow to the ruminant's small intestine to provide an amino acid flow prediction; (305) determining an amino acid requirement of the ruminant based at least in part on the EERQ or MERQ for the ruminant and the quantity of EE or ME, respectively, provided by the amount of the unaltered diet consumed; (306) comparing the amino acid flow prediction to the amino acid requirement of the ruminant to provide an amino acid comparison; (307) determining a rumen microbial peptide-nitrogen or amino acid-nitrogen requirement and a rumen microbial ammonia-nitrogen requirement; (308) comparing peptide- or amino acid-nitrogen supply of the unaltered diet to the microbial peptide-nitrogen or amino acid-nitrogen requirement to provide a peptide- or amino acid-nitrogen comparison; (309) comparing ammonia-nitrogen supply of the unaltered diet to the microbial ammonia-nitrogen requirement to provide an ammonia-nitrogen comparison;

(310) determining, based at least in part on the amino acid comparison, the peptide- or amino acid-nitrogen comparison, and the ammonia-nitrogen comparison, an amount of urea, a protein source, peptides, RPP, RPAA, or any combination thereof, to add to the unaltered diet to provide an amino **acid-balanced** diet in proportion to the EERQ or MERQ of the ruminant; and (311) preparing an adjusted diet, a dietary supplement, or a combination thereof, comprising the amount of urea, protein source, peptides, RPP, RPAA, or combination thereof.

**In** any of the foregoing embodiments, values for energy provided by the unaltered diet, EE requirement, ruminal microbial efficiency, rumen microbial peptide-nitrogen or amino acid-nitrogen requirement, and rumen microbial ammonia-nitrogen requirement may be calculated as shown below. A person of ordinary skill in the art will understand that all numbers shown in the following equations are approximations and further that minor value variations (e.g., ± 10% of the value) also provide acceptable results.

The effective energy (EE) provided by each kilogram of the unaltered diet may be calculated as shown in equation 1: $EE \left(MJ/kg\right) = \left(\left(1.15\times ME\right)-3.84-\left(\left(4.67\times \left(CP\times 0.8/100\right)\right)\right)\right.$ (eq. 1)

With respect to equation 1, ME is metabolizable energy (MJ/kg) of the unaltered diet, and CP is crude protein percentage of the feed. ME may be determined by deducting the energy content of the feces, urine, and greenhouse gas from the gross energy of the unaltered diet; alternatively, ME may be determined based on the acid detergent fiber content of a forage as is known by those of ordinary skill in the art of ruminant feeding. The protein content of the feed may be determined by measuring the nitrogen content of the unaltered diet. Proteins typically contain 16% nitrogen. Thus, CP is determined by multiplying the nitrogen content by 6.25 (or by dividing the nitrogen content by 0.16). Commercially available software may also be used to estimate ME and/or CP content of feed. Feed manufacturers may provide ME and/or CP estimates. The National Research Council (NRC) also provides guidance for estimating dietary ME and/or CP content of feed ingredients (see, e.g., Nutrient Requirements of Dairy Cattle: Seventh Revised Edition, 2001).

The quantity of effective energy or metabolizable energy consumed by the ruminant is based on the average daily intake (kg) of the animal, as shown in equations 2a and 2b: $EE / day = EE \times average daily intake$ (eq. 2a) $ME / day = ME \times average daily intake$ (eq. 2b)

The ruminant's EERQ or MERQ may be calculated using equations 3a and 3b, respectively: $EERQ \left(MJ\right) = \left(MH+\left(PR\times 50\right)+\left(LR\times 56\right)\right) / 1000$ (eq. 3a)

With respect to equation 3a, MH is maintenance heat, PR is protein retention/accretion, and LR is lipid retention/accretion. 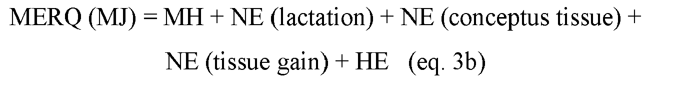 MERQ (MJ) = MH + NE (lactation) + NE (conceptus tissue) + NE (tissue gain) + HE (eq. 3b) **With respect** to equation 3b, MH is maintenance heat, NE is net energy, HE is heat increment or heat generated. HE would be measured as heat given off by an animal. More commonly, it is measured by measuring oxygen intake and carbon dioxide respired and calculating heat of oxygen combustion. Alternatively, NE values may be modeled. NE of lactation, conceptus tissue, and tissue gain are determined as energy of those tissues measured by heat given off when combusted. In some embodiments, EE determinations are preferred because NE tissue gain does not delineate between protein and fat gains.

In some embodiments, equation 3a or 3b is modified to include the energy required for methane gas, i.e., by adding 0.616 × MTHE where MTHE is methane gas energy content (kJ). Alternatively, the energy required for methane generation may be estimated at 12.2% of consumed energy. Thus, the value obtained by equation 3a or 3b may be increased by 12.2% to account for methane generation.

Maintenance heat is the energy required to simply maintain the ruminant without any growth or weight gain. Maintenance heat depends on the ruminant's initial empty body weight (kg) (EBW2) on the measurement day (MD): $EBW 2 = \left(MDwt-ADG\right) \times 0.891$ (eq. 4) $MH = \left({EBW 2}^{∧}0.75\right) \times 420 kJ$ (eq. 5) With respect to equation 4, MDwt is the ruminant's weight at the end of the measurement day and ADG is the ruminant's average daily gain.

The ruminant's energy requirements for protein retention/accretion (PR) and lipid accretion/retention (LR) are based on the ruminant's body protein increase and body lipid increase on measurement day, which is determined from the ruminant's initial empty body weight (EBW2, eq. 4) and final empty body weight (EBW1). The constants in equations 4 and 5 above and the equations below will vary based upon an expected mature weight or frame score of the ruminant (see, e.g., National Resource Councils: Nutrient Requirements of Beef Cattle, Seventh Revised Edition 2000, National Academy Press, Washington DC). As mature body weight increases, the percentage of protein in the body is greater and the percentage of lipid is lower. Gender may also affect the values. The particular constants below assume a 1200-lb mature ruminant: $EBW 1 = MDwt \times 0.891$ (eq. 6) $Body protein for EBW 1 = \left(-2.418+\left(0.235\times EBW1\right)-\left(0.00013\times EBW{1}^{∧}2\right)\right)$ (eq. 7) $Body protein for EBW 2 = \left(-2.418+\left(0.235\times EBW2\right)-\left(0.00013\times {EBW 2}^{∧}2\right)\right)$ (eq. 8) $Protein retention \left(PR\right) = body protein for EBW 1 - body protein for EBW 2$ (eq. 9) $Body lipid for EBW 1 = \left(-0.61+\left(0.037\times EBW1\right)+\left(0.00054\times {EBW 1}^{∧}2\right)\right.$ (eq. 10) $Body lipid for EBW 2 = \left(-0.61+\left(0.037\times EBW2\right)+\left(0.00054\times {EBW 2}^{∧}2\right)\right.$ (eq. 11) $Lipid retention \left(LR\right) = body lipid for EBW 1 - body lipid for EBW 2$ (eq. 12)

Rumen microbial efficiency (MOEFF) is a function of ruminal dilution rate (DR). In some embodiments, MOEFF based on starch (non-structural carbohydrates, NSC), nondigestible fiber (NDF), and protein (CP) content of the ruminant's diet is calculated according to the sum of equations 13-15. For equations 13-15, microbial mass in the rumen is distributed into two pools, the solids associated (SA) and liquid associated (LA) pools, with values typically being 0.80 for SA and 0.20 for LA. The MOEFF and the outflow of the SA and LA associated micro-organisms is affected by the DR of the specific fractions, that is the SA-associated organisms flow according to the solid DR and the LA-associated organisms flow at the liquid DR. The microbial protein yield (grams) is calculated by multiplying each of the MOEFF values by the mass (kg) of the diet component (starch, NDF, protein) consumed per day that is fermented in the rumen. The flow of metabolizable microbial true protein is calculated by multiplying microbial protein flow × true protein content (MTP) and the absorption coefficient of the true protein (MTPD). 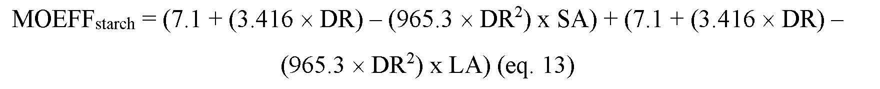 MOEFFstarch = (7.1 + (3.416 × DR) - (965.3 × DR2) x SA) + (7.1 + (3.416 × DR) - (965.3 × DR2) x LA) (eq. 13) 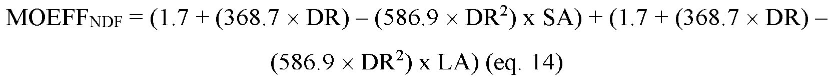 MOEFFNDF = (1.7 + (368.7 × DR) - (586.9 × DR2) x SA) + (1.7 + (368.7 × DR) - (586.9 × DR2) x LA) (eq. 14) 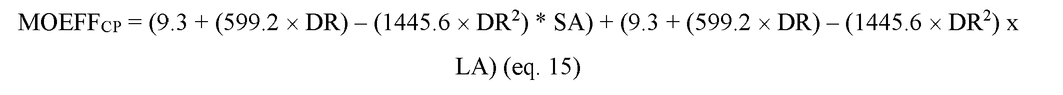 MOEFFCP = (9.3 + (599.2 × DR) - (1445.6 × DR2) * SA) + (9.3 + (599.2 × DR) - (1445.6 × DR2) x LA) (eq. 15) 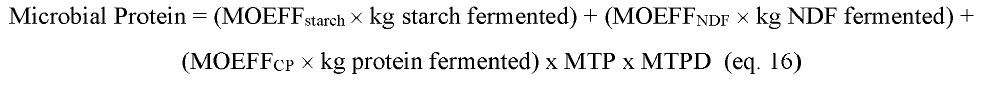 Microbial Protein = (MOEFFstarch × kg starch fermented) + (MOEFFNDF × kg NDF fermented) + (MOEFFCP × kg protein fermented) x MTP x MTPD (eq. 16)

Dilution rate is entered as the fractional DR; e.g., if DR is 5%, 0.05 is entered into the equations. The percentage of micro-organisms associated with the solid and liquid pools is entered as a fraction; e.g., if SA is 80%, 0.80 is entered into the equations. Although there are individual variations in DR, on average, DR for lactating ruminants , such as dairy cows, is 5-6% per hour, DR for cattle on pasture is 2.5%/hour, and for growing animals confined in a feedlot DR is 5%/hour on diets without roughage, and 4%/hour on diets with roughage. In like manner, although variation exists, typically the distribution of organisms in the rumen is 80% SA and 20% LA. The proportion of true protein (MTP) in the microbial crude protein is assumed to be 85%, which is entered as a fraction, i.e. 0.85, and the digestion and absorption of microbial true protein (MTPD) from the intestine is assumed to be 85%, which is also entered as a fraction, i.e., 0.85. The quantity of metabolizable microbial essential AA supplied to the animal is calculated as MTP multiplied by the percentage of the essential AA in microbial protein. The typical composition of ruminal bacteria is presented in Table 1 below.

**Table 1**

| **Amino Acids** | **G/100 G Of Protein** |
|---|---|
| Methionine | 2.68 |
| Lysine | 8.20 |
| Histidine | 2.69 |
| Phenylalanine | 5.16 |
| Tryptophan | 1.63 |
| Threonine | 5.59 |
| Leucine | 7.51 |
| Isoleucine | 5.88 |
| Valine | 6.16 |
| Arginine | 6.96 |
| Source: Clark et al. 1992. Microbial protein synthesis and flows of nitrogen fractions to the duodenum of dairy cows. J. Dairy Science 75:2304 | |

In some embodiments, rumen microbial peptide- or amino acid-nitrogen requirement and rumen microbial ammonia-nitrogen requirement are based on MOEFF and bacterial protein synthesized. Microbial protein synthesized is calculated as shown above in equations 13-16. Protein typically contains 16% nitrogen as discussed above. Fiber fermenting micro-organisms found in the rumen utilize ammonia nitrogen as the primary source of nitrogen for protein synthesis whereas starch fermenting micro-organisms derive two-thirds of the required nitrogen in the form of peptide nitrogen and one-third in the form of ammonia nitrogen. Protein fermenting micro-organisms utilize exclusively peptide nitrogen as the preferred form of nitrogen for protein synthesis. However, because there is only 80% use efficiency for peptide nitrogen, the calculated peptide nitrogen requirement is adjusted according to the equation absolute peptide-nitrogen requirement/0.8 equaling actual peptide nitrogen requirement.

In some embodiments, protein provided by the unaltered diet is determined by measuring the amount of protein in all feed ingredients, and the rate of protein degradation in the rumen for each feed ingredient is determined. Alternatively, values for rate of protein degradation may be found in the literature, e.g., National Research Council publications. As protein is fermented, it is converted from protein to peptides to amino acids to ammonia. The rumen degradable protein (RDP) of feedstuffs is determined by multiplying the potentially degradable protein amount by the rate of protein degradation divided by the sum of the rate of protein degradation and rate of protein passage from the rumen. The rumen undegradable protein (RUP) content of feedstuffs is calculated as either the inverse of RDP (i.e., Total Protein - RDP) or as protein in the ingredient minus the indigestible protein in the ingredient minus the rumen degradable protein in the ingredient. The amino acid flow to the small intestine is calculated as the mass of RUP multiplied by the fraction of each amino acid in the protein. The amounts of NDF and starch fermented in the rumen are determined by the method used to calculate RDP, as described above. The requirement of peptide-nitrogen for starch-fermenting bacteria is subtracted from the RDP pool. The amount of ammonia required by starch and NDF fermenting bacteria is subtracted from the RDP/ammonia pool. These calculations determine whether peptide and ammonia-nitrogen requirements are met, with rumen soluble amino acids supply at least a portion of the peptide-nitrogen requirement. If not, then proteins, peptides, amino acids, urea, or any combination thereof are used to meet microbial requirements for RDP and RDN.

The rumen outflow of amino acids to the true stomach and small intestine is calculated as the sum of amino acids flowing from microbial protein and from dietary protein (RUP). The flow of individual AA derived from microbial protein is calculated as total microbial protein multiplied by the fraction of each amino acid in microbial protein. Dietary amino acid flow is calculated as RUP multiplied by the fraction of each amino acid in the RUP. The flow of absorbable AA is calculated by multiplying total amino acid by 0.85, to account for an intestinal absorption efficiency of 85% for AA. The AA absorbed from the small intestine are considered metabolizable AA. Metabolizable amino acids are compared to the amino acid requirement to assess whether amino acid excesses or deficiencies exist. If deficiencies or imbalances exist, then proteins are adjusted in their dietary percentage, new proteins are added, rumen protected amino acids are included, or urea is added to balance the predicted supply of metabolizable AA in relation to AA requirement.

Similar calculations are performed for lactating ruminants. The amino acid requirement for maintenance and growth is as described for non-dairy ruminants. Additionally, the amino acids used by the mammary tissue to form and secrete milk proteins are included in the estimate of AA requirement. For pregnant females, the AA requirement for fetal growth are further included in the estimate of AA requirement.

The calculations for amino acid requirements are generally as described by NRC (2000), as follows: $Maintenance MPAAi = AATISSi \times 0.01 \times \left(FPN+\left(\left(UPA+SPA\right)\times 0.67\right)/EAAMi\right)$

Where: AATISSi is amino acid composition of tissue as presented in the table below. MPAAi is metabolizable requirement for the ith absorbed amino acid, g/day. EAAMi is efficiency of use of the ith amino acid for maintenance as presented in the table. $Growth RPAAi = AATISSi \times 0.01 \times \left(NPg+MPmm\times 0.28908\right) / EAAGi$

Where: AATISSi is amino acid composition of tissue and EAAGi is efficiency of amino acid use, which is assumed to be .29. RPAAi is growth requirement for the ith absorbed amino acid, g/day. NPg is net protein required for growth, g/day. $Lactation LPAAi = \left(AALACTi\times 0.01\times \left(LP\times 0.65\right)\right) / EAALi$

Where: AALACTi is the ith amino acid content of milk true protein, g/100g, as presented in the table. EAALi is efficiency of use of the ith amino acid for milk protein formation. LPAAi is metabolizable requirement for lactation for the ith absorbed amino acid, g/day. 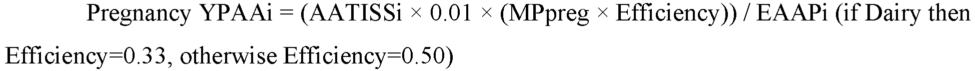 Pregnancy YPAAi = (AATISSi × 0.01 × (MPpreg × Efficiency)) / EAAPi (if Dairy then Efficiency=0.33, otherwise Efficiency=0.50)

Where: AATISSi is amino acid composition of tissue and EAAPi is efficiency of use of the ith amino acid for gestation, g/g. PPAAi is metabolizable requirement for gestation for the ith absorbed amino acid, g/day.

**Table 2**

| **Amino acid** | **Tissue, g/100 g of protein** | **Milk, g/100 g of protein** | **Maintenance Efficiency of Use (EAAM)** | **Lactation Efficiency of Use (EAAL)** | **Pregnancy Efficiency of Use (EAAP)** |
|---|---|---|---|---|---|
| Met | 1.97 | 2.71 | .85 | .98 | .85 |
| Lys | 6.37 | 7.62 | .85 | .88 | .85 |
| His | 2.47 | 2.74 | .85 | .90 | .85 |
| Phe | 3.53 | 4.75 | .85 | 1.00 | .85 |
| Trp | 0.49 | 1.51 | .85 | .85 | .85 |
| Thr | 3.90 | 3.72 | .85 | .83 | .85 |
| Leu | 6.70 | 9.18 | .66 | .72 | .66 |
| Ile | 2.84 | 5.79 | .66 | .62 | .66 |
| Val | 4.03 | 5.89 | .66 | .72 | .66 |
| Arg | 3.30 | 3.40 | .85 | .85 | .66 |

In any of the foregoing embodiments, the adjusted diet, dietary supplement, or combination thereof may provide grams of essential amino acids per megajoule of effective energy within ranges of: Lys - 0.2-2, 0.2-1, 0.3-0.9, or 0.4-0.8 g/MJ; Met - 0.05-1, 0.05-0.5 0.1-0.3, or 0.12-0.25 g/MJ; His - 0.1-1, 0.1-0.5, 0.1-0.4, or 0.15-0.35 g/MJ; Phe - ≥ 0.1 ≥ 0.2, 0.2-2, 0.2-1.5, 0.2-1, or 0.2-0.5 g/MJ; Thr - ≥ 0.1, ≥ 0.2, ≥ 0.25, 0.25-2, 0.25-1.5, 0.25-1, or 0.25-0.5 g/MJ; Leu - ≥ 0.4, ≥ 0.45, 0.45-5, 0.45-2.5, 0.45-2, or 0.45-1 g/MJ; Val - ≥ 0.2, ≥ 0.25, 0.25-2, 0.25-1.5, 0.25-1, or 0.25-0.5 g/MJ; Arg - 0.2-2, 0.3-1, 0.4-0.9, or 0.4-0.85 g/MJ; Ile - ≥ 0.1 ≥ 0.2, 0.2-2, 0.2-1, 0.2-0.5, or 0.2-0.4 g/MJ; Trp - ≥ 0.02, ≥ 0.03, 0.03-0.3, 0.03-0.2, 0.03-0.1, or 0.03-0.07 g/MJ, or any combination thereof for growing animals. In one embodiment, the adjusted diet, dietary supplement, or combination thereof provides grams of essential amino acids per megajoule of effective energy within ranges of Lys 0.2-2, Met 0.05-1, His 0.1-1, Phe > 0.2, Thr > 0.2, Leu > 0.4, Val > 0.2, Arg 0.2-2, Ile > 0.2, Trp > 0.03, or any combination thereof for growing animals. In an independent embodiment, the adjusted diet, dietary supplement, or combination thereof provides grams of essential amino acids per megajoule of effective energy within ranges of Lys 0.4-0.8, Met 0.12-0.25, His 0.15-0.35, Phe ≥ 0.2, Thr ≥ 0.25, Leu ≥ 0.45, Val ≥ 0.25, Arg 0.4-0.8, Ile ≥ 0.2, Trp ≥ 0.03, or any combination thereof for growing animals.

In any of the foregoing embodiments, the adjusted diet, dietary supplement, or combination thereof may provide grams of essential amino acids per megacalorie of metabolizable energy within ranges of: Lys - 1-10, 1-5, 2-4, 2.5-4, or 2.9-4 g/Mcal; Met - 0.4-2.5, 0.5-2, 0.5-1.5, or 0.9-1.2 g/Mcal; His - 0.5-3, 0.5-2, 1-2, or 1.2-1.5 g/Mcal; Phe - ≥ 1.5, ≥ 1.7, 1.5-15, 1.7-10, 1.7-5, or 1.7-2.5 g/Mcal; Thr - ≥ 1, ≥ 1.5, 1-10, 1.5-10, 1.5-5, or 1.5-2.5 g/Mcal; Leu - ≥ 3, ≥ 3.5, 3.5-35, 3.5-20, 3.5-10, or 3.5-5 g/Mcal; Val - ≥ 1.5, ≥ 2, 2-20, 2-10, 2-5, or 2-4 g/Mcal; Arg - ≥ 1.5, ≥ 1.8, 1.8-18, 1.8-10, or 1.8-5 g/Mcal; Ile - ≥ 1.5, ≥ 1.6, 1.6-15, 1.6-10, or 1.6-5 g/Mcal; Trp - ≥ 0.3, ≥ 0.4, 0.4-4, 0.4-3, or 0.4-2 g/Mcal; or any combination thereof for lactating animals. Alternatively, the adjusted diet, dietary supplement, or combination thereof may provide grams of essential amino acids per megajoule of metabolizable energy within ranges of: Lys - 0.2-2.5, 0.2-1.2, 0.4-1, 0.6-1, or 0.7-1 g/MJ; Met 0.1-0.6, 0.1-0.5, 0.1-0.4, or 0.2-0.3 g/MJ; His - 0.1-1, 0.1-0.7, 0.1-0.5, 0.2-0.5, or 0.25-0.35 g/MJ; Phe - ≥ 0.3, ≥ 0.4, 0.3-4, 0.4-2.5, 0.4-1.2, or 0.4-0.6 g/MJ; Thr -≥ 0.2, ≥ 0.35, 0.2-2.5, 0.35-2.5, 0.35-1.2, or 0.35-0.6 g/MJ; Leu - ≥ 0.7, ≥ 0.84, 0.8-8, 0.8-5, 0.8-2.5, or 0.84-1.2 g/MJ; Val - ≥ 0.3, ≥ 0.47, 0.5-5, 0.5-2.5, 0.5-1.2, or 0.5-1 g/MJ; Arg -≥ 0.3, ≥ 0.43, 0.4-4, 0.4-2.5, or 0.43-1.2 g/MJ; Ile - ≥ 0.3, ≥ 0.38, 0.3-4, 0.35-2.5, or 0.38-1.2 g/MJ; Trp -≥ 0.07, ≥ 0.09, 0.09-1, 0.09-0.7, or 0.09-0.5 g/MJ; or any combination thereof for lactating animals. In one embodiment, the adjusted diet, dietary supplement, or combination thereof may provide grams of essential amino acids per megajoule of metabolizable energy within ranges of: Lys - 0.2-2.5; Met 0.1-0.6; His - 0.1-1; Phe - ≥ 0.3; Thr -≥ 0.2; Leu - ≥ 0.7; Val - ≥ 0.3; Arg -≥ 0.3; Ile - ≥ 0.3; Trp -≥ 0.07; or any combination thereof for lactating animals. In an independent embodiment, the adjusted diet, dietary supplement, or combination thereof may provide grams of essential amino acids per megajoule of metabolizable energy within ranges of: Lys 0.7-1, Met 0.2-0.3, His 0.25-0.35, Phe ≥ 0.4, Thr ≥ 0.35, Leu ≥ 0.84, Val ≥ 0.47, Arg ≥ 0.43, Ile ≥ 0.38, Trp ≥ 0.09, or any combination thereof for lactating animals.

### III. Methods of Using the Dietary Supplement or Adjusted Diet

A ruminant can be administered a diet (an "unaltered diet") and an amount of a dietary supplement prepared as disclosed herein. The amount of the dietary supplement may be effective to mitigate an amino acid deficiency of the ruminant's unaltered diet.

A ruminant can be administered an amount of an adjusted diet prepared as disclosed herein. The amount of the adjusted diet can be effective to mitigate an amino acid deficiency of the ruminant's unaltered diet. The adjusted diet may replace the ruminant's unaltered diet. Alternatively, the ruminant may be administered an amount of the adjusted diet in addition to an amount of the unaltered diet.

A ruminant can be administered an amount of a dietary supplement and an amount of an adjusted diet as disclosed herein. The amount of the dietary supplement may mitigate, for example, one amino acid deficiency while the amount of the adjusted diet mitigates another amino acid deficiency. Alternatively, the dietary supplement and adjusted diet may each mitigate a portion of an amino acid deficiency, and in combination completely or substantially completely mitigate the deficiency.

The amino acid deficiency may be a deficiency in one or more essential amino acids. The dietary supplement and/or adjusted diet may be administered to the ruminant periodically at time intervals determined to be effective, typically at least daily. The ruminant may be a bovine, an ovine, or a caprine. In particular, the ruminant is a bovine.

Administering may comprise administering the dietary supplement directly or indirectly to the ruminant. Direct administration includes, for example, orally administering a dietary supplement bolus to the ruminant. The bolus may be a solid (e.g., a capsule or tablet) or a liquid formulation. Indirect administration includes combining the dietary supplement with the ruminant's unaltered diet or water, whereby the ruminant consumes the dietary supplement with the unaltered diet or water. An adjusted diet is administered to the ruminant in place of the unaltered diet or in addition to the unaltered diet, whereby the ruminant consumes the adjusted diet. In some cases, the ruminant is administered a dietary supplement and an adjusted diet. In such cases, the dietary supplement may be administered as described above or the dietary supplement may be combined with the adjusted diet.

Advantageously, administering (i) the amount of the adjusted diet, (ii) the amount of the unaltered diet and the amount of the dietary supplement, or (iii) the amount of the adjusted diet and the amount of the dietary supplement to the ruminant may: increase the ruminant's average daily gain; decrease the ruminant's dry matter intake; increase the ruminant's feed efficiency; decrease the ruminant's feed-to-gain ratio; decrease the ruminant's cost of gain; decrease the ruminant's gas emission (e.g., methane, nitrous oxide, carbon dioxide, or any combination thereof); reduce the ruminant's age of reproductive development; improve the ruminant's reproductive success; increase the ruminant's carcass weight; increase the ruminant's yield and/or quality grade; increase the ruminant's carcass dressing percentage; or any combination thereof; as compared to a ruminant that did not receive the adjusted diet and/or dietary supplement. In some embodiments, the ruminant is a dairy ruminant and administering the diet and the amount of the dietary supplement to the ruminant increases the ruminant's milk yield, increases the ruminant's milk efficiency, increases the ruminant's milk components (i.e., increases the amount of milk fat, milk protein, milk sugar, and the like obtained from the ruminant), reduces the ruminant's milk somatic cell count, or any combination thereof compared to a dairy ruminant that did not receive the adjusted diet and/or dietary supplement.

The method may further include administering a growth promotant to the ruminant. The growth promotant may be a hormone, an antimicrobial, a direct-fed microbial, a beta agonist, a plant extract, or any combination thereof. In some embodiments, the antimicrobial is an ionophore, an antibiotic, an antifungal agent, an antiviral agent, an antiparasitic agent, or any combination thereof.

### IV. Computer-implementation

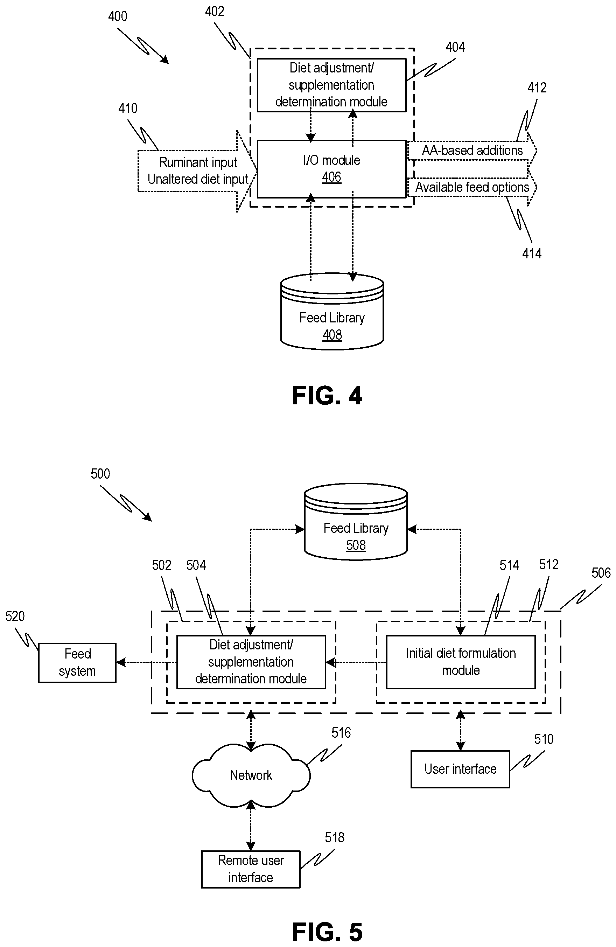

FIG. 4 illustrates an exemplary configuration 400 of a computing device 402 that can be used to perform any of the methods (or parts thereof) disclosed herein. For example, the computing device 402 includes an input/output module 406 and a determination module 404. The determination module 404 can be configured to determine an amount of urea, a protein source, peptides, rumen-protected peptides, rumen-protected amino acids, or any combination thereof, to add to an initial ruminant diet to provide an amino acid-balanced diet that satisfies a ruminant's amino acid requirement using any of the above described equations or algorithms. Alternatively or additionally, in some embodiments, the determination module 404 can be configured to determine an amino-acid-balanced diet formulation that satisfies a ruminant's amino acid requirement, for example, by determining adjustment(s) to an initial diet (or an adjusted diet) and/or dietary supplement(s) to add to an initial diet.

The computing device 402 can receive one or more signals 410 at the input/output module 406 that convey input data or information used by the determination module 404 in executing the disclosed methods. For example, signal(s) 410 can provide data regarding the target ruminant (e.g., body weight, species, type (such as growing or lactating), etc.) and/or data regarding the initial ruminant diet (e.g., composition details). Other input data or information is also possible, as otherwise described herein. **In** some embodiments, signal(s) 410 can be sent to the computing device 402 in response to input by a user via a user interface (e.g., a display and keyboard associated with the computing device 402 or a remote computing device). Alternatively or additionally, signal(s) 410 can originate at another computing device or module that determines a preliminary diet formulation (e.g., module 514 in FIG. 5).

**In** some embodiments, signal(s) 410 can specify high-level information (e.g., name of an available feed mix) regarding the initial ruminant diet rather than composition details. **In** such embodiments, the computing device 402 can retrieve composition details based on the high-level information, for example, by sending an inquiry to and receiving a response from feed library 408. Feed library 408 can be a database or other data storage element (e.g., memory) that stores available feed options (e.g., commercially-available feed mixes and/or dietary supplements for ruminants) and associated composition details. **In** some embodiments, feed library 408 can be a separate structure that computing device 402 accesses over a wired or wireless connection. Alternatively, in some embodiments, feed library 408 can be part of computing device 402, for example, stored on internal memory.

Using the input data or information, the determination module 404 can determine an amino-acid-balanced diet formulation that satisfies a ruminant's amino acid requirement. For example, the determination module 404 can determine an amount of urea, a protein source, peptides, rumen-protected peptides, rumen-protected amino acids, or any combination thereof, to add to an initial ruminant diet to provide an amino acid-balanced diet. **In** some embodiments, the computing device 402 can generate and send one or more first output signals 412 indicative of the determined amounts. For example, first output signal(s) 412 can cause a user interface (e.g., associated with the computing device 402 or a remote computing device) to display the determined amounts. A person interacting with the user interface can then use the knowledge of the determined amounts to alter the diet for the ruminant, for example, by selecting an additional dietary component or a dietary supplement, that satisfies the determined amounts.

**In** some embodiments, the determination module 404 can select one or more available options for feed and/or dietary supplement that satisfy the determined amino acid amounts. For example, the determination module 404 can send an inquiry to and receive a response from feed library 408 regarding commercially-available feed and/or dietary supplement options that have amino acid amounts that meet the determined formulation. In some embodiments, the computing device 402 can generate and send one or more second output signals 414 indicative of the selected options, which output signal(s) may be in addition to or in place of first output signal(s) 412. For example, second output signal(s) 414 can cause the user interface (e.g., associated with the computing device 402 or a remote computing device) to display the selected available options. The person interacting with the user interface can then use the list presented by the computing device 402 to select an available feed or dietary supplement option. For example, the person can select an option or options from the presented list that have the lowest cost, while ensuring that the determined amino acid amounts for satisfying the ruminant's amino acid requirement are met.

Alternatively or additionally, in some embodiments, output signal(s) 412 or output signal(s) 414 can be directed to a feed system that administers or otherwise provides feed to the ruminant. The output signal(s) 412 and/or output signal(s) 414 can control the feed system to automatically adjust the feed administered to the ruminant, for example, by administering the determined amount of adjusted diet and/or dietary supplement in addition to or in place of the initial diet.

FIG. 5 illustrates an exemplary configuration 500 of a computerized system 506 that can be used to formulate a diet for a ruminant by performing any of the methods (or parts thereof) disclosed herein. For example, the computerized system 506 includes an initial diet formulation (IDF) module 514 and a diet adjustment/supplementation determination (DASD) module 504. The IDF module 514 can determine an initial diet for the ruminant. For example, the IDF module 514 can predict dietary requirements, feed utilization, animal performance, and/or nutrient excretion for ruminants and can determine an initial diet that meets those requirements. For example, the IDF module 514 can employ the prediction model provided by the Cornell Net Carbohydrate and Protein System (CNCPS, Version 6.5.5) or commercial software such as but not limited to AMTS software products (e.g., AMTS.Farm.Cattle, AMTS.Farm.Small Ruminants, or AMTS.GrowingCattle, sold by Agricultural Modeling and Training Systems, LLC, Groton, NY), RUM&N software products (e.g., NDS Professional or Performilk, sold by RUM&N SAS, Italy), The Consulting Nutritionist software (sold by Dalex Livestock Solutions, LLC, Los Angeles, CA), and Fabermatica software products (PluriMix or DinaMilk, sold by Fabermatica, Italy).

As shown in FIG. 5, the IDF module 514 can communicate with a feed library 508, which can be a database or other data storage element (e.g., memory) that stores feed options (e.g., commercially-available feed mixes and/or dietary supplements for ruminants) and associated composition or dietary details. In some embodiments, feed library 508 can be a separate structure from computerized system 506. IDF module 514 can thus accesses the feed library 508 over a wired or wireless connection. Alternatively, in some embodiments, feed library 508 can be part of computerized system 506, for example, stored on internal memory.

For example, the IDF module 514 can send an inquiry to and receive response from feed library 508 regarding commercially-available feed and/or dietary supplement options meet the predicted requirements. Based on the available options, the IDF module 514 can construct an initial diet formulation, which formulation can be output as data or signals from the IDF module 514, for example, as an output data file stored in memory of the computerized system 506, as an output signal to the DASD module 504, as a signal to user interface 510 associated with computerized system 506 or a remote user interface 518, or any combination thereof.

Using the initial diet from the IDF module 514 and any of the above described equations or algorithms, the DASD module 504 can determine an amount of urea, a protein source, peptides, rumen-protected peptides, rumen-protected amino acids, or any combination thereof, to add to an initial ruminant diet to provide an amino acid-balanced diet that satisfies a ruminant's amino acid requirement. Alternatively or additionally, in some embodiments, the DASD module 504 can be configured to determine an amino-acid-balanced diet formulation that satisfies a ruminant's amino acid requirement, for example, by determining adjustment(s) to an initial diet (or an adjusted diet) and/or dietary supplement(s) to add to an initial diet. In some embodiments, the DASD module 504 can select one or more available options for feed and/or dietary supplement that satisfy the determined amino acid amounts. For example, the DASD module 504 can send an inquiry to and receive a response from feed library 508 regarding commercially-available feed and/or dietary supplement options that have amino acid amounts that meet the determined formulation.

In some embodiments, IDF module 514 and DASD module 504 can be part of or operate within a single computing device. For example, IDF module 514 can be a first software program operating on a computer, and the DASD module 504 can be a second software program operating on the same computer. In such configurations, the computerized system 506 may comprise a single computing device, and IDF module 514 and DASD module 504 can operate separately from each other within computerized system 506. DASD module 504 can be configured to receive an output (e.g., one or more output signal(s), data files, or other information) from IDF module 514, and DASD module 504 and IDF module 514 can share internal resources, for example, memory and/or processing.

Alternatively, in some embodiments, IDF module 514 and DASD module 504 can be part of or operate within different computing devices. For example, IDF module 514 can be a first software program operating on a first computer (e.g., dashed line 512), and the DASD module 504 can be a second software program operation on a second computer (e.g., dashed line 502). In such configurations, the computerized system 506 may comprise separate computing devices for the IDF module 514 and the DASD module 504. DASD module 504 and IDF module 514 would thus have their own separate computing resources, e.g., memory, processor, etc. Communication between the separate computing devices can occur over a wired or wireless connection, for example, via a direct connection between the computing devices or via a network connection, such that DASD module 504 can receive an output (e.g., one or more output signal(s), data files, or other information) from IDF module 514.

In some embodiments, a user interface 510 is associated with computerized system 506 and allows a person or other computerized system to interact with modules 504, 514, for example, to provide input information or data thereto and/or to receive output information or data therefrom. Alternatively or additionally, a user interface 518 can be provided that is remote from the computerized system 506 and allows a person or other computerized system to remotely interact with modules 504, 514 via intervening network 516. The network 516 can be an Internet area network (IAN), a wide area network (WAN), a local area network (LAN) connected to a WAN, or any other network configuration or combinations thereof. It should be appreciated that the configuration 500 illustrated in FIG. 5 has been simplified and that many more networks and networking devices can be utilized to interconnect the various systems disclosed herein.

In some embodiments, user interface 510 or 518 can be a web-based graphical user interface (GUI) or an application programmatic interface (API), for example, when a person or other computerized system programmatically interacts with DASD module 504 and/or IDF module 514. For example, the API of user interface 510 can function as an endpoint that allows programmatic integration of the computerized system 506 into existing feed operation systems. When user interface 510 or 518 is configured as an API for the computerized system 506, a request to modules 504 and/or 514 over network 516 (or any other network) can be initiated using, for example, an API request. For purposes of simplicity, network service requests will be generally described herein as API requests, but it is understood that other network service requests can be made.

An API request is a programmatic interface to a defined request-response message system, typically expressed in JSON or XML, which is exposed via the web - most commonly by means of an HTTP-based web server. Thus, in certain implementations, an API can be defined as a set of Hypertext Transfer Protocol (HTTP) request messages, along with a definition of the structure of response messages, which can be in an Extensible Markup Language (XML) or JavaScript Object Notation (JSON) format. The API can specify a set of functions or routines that perform an action, which includes accomplishing a specific task or allowing interaction with a software component. When a network service receives the API request from a client device or from the host computer, the network service can generate a response to the request and send the response to the endpoint identified in the request. For example, the API request may include inputs regarding target ruminant (e.g., body weight, species, type (such as growing or lactating), etc.) and/or data regarding the initial ruminant diet (e.g., composition details), and the API can return to the requestor or another computing device data regarding the determined amount of urea, protein source, peptides, rumen-protected peptides, rumen-protected amino acids, or any combination thereof, and/or data regarding the determined amount of adjusted diet and/or dietary supplement in addition to or in place of the initial diet.

In some embodiments, determinations by the DASD module 504 can be output as data or signals, for example, as a signal to user interface 510 associated with computerized system 506 or a remote user interface 518, as an output data file stored in memory of the computerized system 506, as a signal to feed system 520, or any combination thereof. For example, in some embodiments, the computerized system 506 can send one or more output signals to control feed system 520 to automatically adjust the feed administered to the ruminant, for example, by administering the determined amount of adjusted diet and/or dietary supplement in addition to or in place of the initial diet.

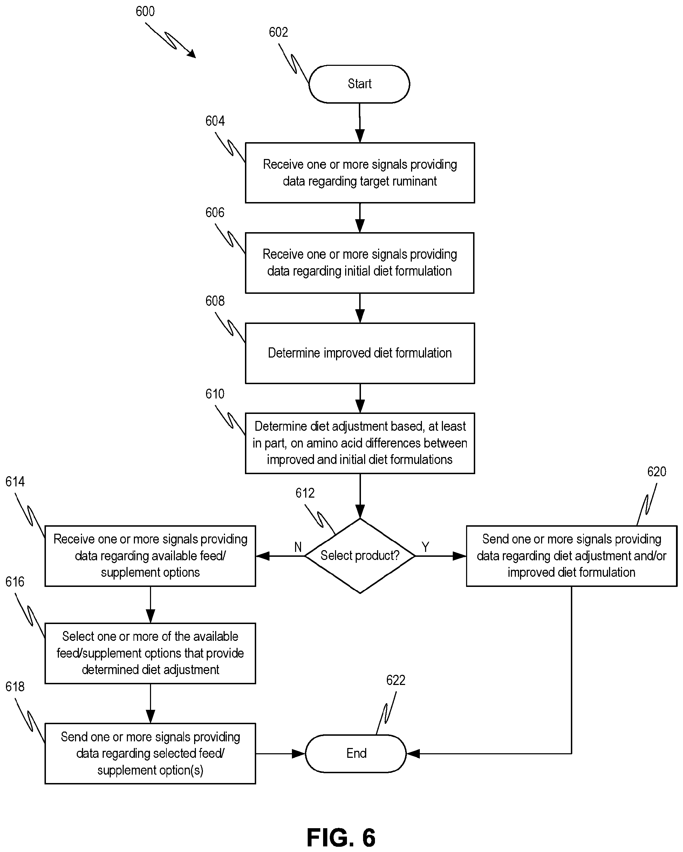

FIG. 6 is a simplified process flow diagram of a computer-implemented method 600 for formulating a diet for ruminant. The method 600 can initiate at 602 and proceed to process block 604, where one or more first signals are received. In some embodiments, the first signal(s) can be received by any of the above-described computing devices, computerized systems, or subcomponents thereof (e.g., I/O module 406, determination module 404, IDF module 514 or DASD module 504). For example, the first signal(s) received at process block 604 can convey data regarding a target ruminant, such as, but not limited to, body weight, species, type (such as growing or lactating), etc. Other data or information regarding the ruminant is also possible, as otherwise described herein. In some embodiments, the first signal(s) can be sent from a user interface (e.g., user interface 510 or 518) or from a computing device, computerized system, or subcomponent thereof (e.g., IDF module 514).

The method 600 can further include process block 606, where one or more second signals are received. In some embodiments, the second signal(s) can be received by any of the above-described computing devices, computerized systems, or subcomponents thereof (e.g., I/O module 406, determination module 404, or DASD module 504). For example, the second signal(s) received at process block 606 can convey data regarding an initial ruminant diet, such as, but not limited to, feed quantity, composition details, etc. Other data or information regarding the ruminant diet is also possible, as otherwise described herein. In some embodiments, the second signal(s) can be sent from a user interface (e.g., user interface 510 or 518) or from a computing device, computerized system, or subcomponent thereof (e.g., IDF module 514).

The method 600 can further include process block 608, where an improved diet formulation can be determined. For example, the improved diet formulation can be an amino acid-balanced diet that satisfies a ruminant's amino acid requirement. The amino-acid-balanced diet can be determined using any of the above described equations or algorithms. In some embodiments, the improved diet formulation can be determined by an appropriate module of a computing device (e.g., determination module 404) or computerized system (e.g., DASD module 504).

The method 600 can further include process block 610, where a diet adjustment can be determined. For example, the determined diet adjustment can include an amount of urea, a protein source, peptides, rumen-protected peptides, rumen-protected amino acids, or any combination thereof, to add to the initial ruminant diet to provide the amino-acid-balanced diet. The determination of the diet adjustment can be based, at least in part, on amino acid differences between improved and initial diet formulations. The diet adjustment can be determined using any of the above described equations or algorithms. In some embodiments, the diet adjustment can be determined by an appropriate module of a computing device (e.g., determination module 404) or computerized system (e.g., DASD module 504).

The method 600 can further include decision block 612, where it is determined if a feed product or dietary supplement should be selected. If product selection is not desired, the method can proceed from decision block 612 to process block 620, where one or more third signals are generated and sent. In some embodiments, the third signal(s) can be generated by any of the above-described computing devices, computerized systems, or subcomponents thereof (e.g., I/O module 406, determination module 404, or DASD module 504). For example, the third signal(s) generated at process block 612 can convey data regarding the determined diet adjustment and/or the improved diet formulation. In some embodiments, the third signal(s) can be sent to a user interface (e.g., user interface 510 or 518) or to another computing device, computerized system, or subcomponent thereof. After process block 620, the method can optionally terminate at 622.

If product selection is desired, the method can proceed from decision block 612 to process block 614, where one or more fourth signals are received. In some embodiments, the fourth signal(s) can be received by any of the above-described computing devices, computerized systems, or subcomponents thereof (e.g., I/O module 406, determination module 404, or DASD module 504). For example, the fourth signal(s) received at process block 614 can convey data regarding available feed options (e.g., commercially-available feed mixes and/or dietary supplements for ruminants) and associated composition details. In some embodiments, the fourth signal(s) can be sent from an internal data storage device (e.g., a memory associated with computing device 402 or computerized system 506), from an external data storage device (e.g., feed library 408 or 508), or from another computing device, computerized system, or subcomponent thereof (e.g., IDF module 514).

The method 600 can further include process block 616, where one or more available feed mixes and/or dietary supplements are selected to provide the determined diet adjustment. In some embodiments, the selections can be made by an appropriate module of a computing device (e.g., determination module 404) or computerized system (e.g., DASD module 504). For example, the selection can be made from available options received at process block 614 by comparing composition details to the requirements of the previously determined diet adjustment. Alternatively or additionally, in some embodiments, the options received at process block 614 may be only those options that meet a pre-specified composition criteria, and the selection at process block 616 may order those options into a list for subsequent consideration and selection by a user or other computerized system.

The method 600 can further include process block 618, where one or more fifth signals are generated and sent. In some embodiments, the fifth signal(s) can be generated by any of the above-described computing devices, computerized systems, or subcomponents thereof (e.g., I/O module 406, determination module 404, or DASD module 504). For example, the fifth signal(s) generated at process block 618 can convey data regarding the selected feed and/or dietary supplement options. In some embodiments, the fifth signal(s) can be sent to a user interface (e.g., user interface 510 or 518) or to another computing device, computerized system, or subcomponent thereof. For example, the fifth signal(s) can cause display of a list of the selected options to a user for consideration and selection. The person interacting with the display can then use the to select an available feed or dietary supplement option. For example, the person can select an option or options from the presented list that have the lowest cost, while ensuring that the determined amino acid amounts for satisfying the ruminant's amino acid requirement are met. Alternatively or additionally, in some embodiments, the fifth signal(s) can cause an automated feed system, which administers or otherwise provides feed to the ruminant, to automatically alter the feed composition based on the selected options. After process block 620, the method can optionally terminate at 622.

Although FIG. 6 illustrates a particular order for blocks 602-622, embodiments of the disclosed subject matter are not limited thereto. Indeed, in certain embodiments, the blocks may occur in a different order than illustrated or simultaneously with other blocks. For example, the receipt of signal(s) regarding the target ruminant at process block 604 may occur after or at a same time as the receipt of signal(s) regarding the initial diet formulation at process block 606. Moreover, although blocks 602-622 are shown separately or in the alternative, embodiments of the disclosed subject matter are not limited thereto. For example, in some embodiments, process blocks 614-618 can be performed together with process block 620 rather than in the alternative.

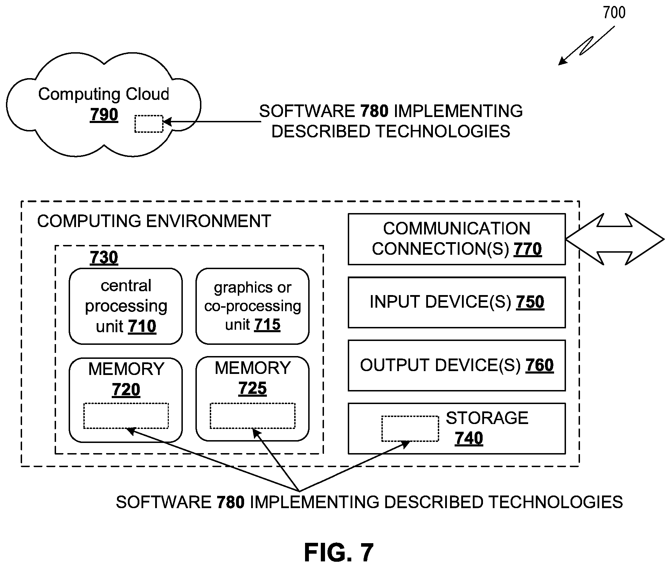

FIG. 7 depicts a generalized example of a suitable computing environment 700 in which the described innovations may be implemented. The computing environment 700 is not intended to suggest any limitation as to scope of use or functionality, as the innovations may be implemented in diverse general-purpose or special-purpose computing systems. For example, the computing environment 700 is any of a variety of computing devices (e.g., desktop computer, laptop computer, server computer, tablet computer, etc.).

The computing environment 700 includes one or more processing units 710, 715 and memory 720, 725. In FIG. 7, this basic configuration 730 is included within a dashed line. The processing units 710, 715 execute computer-executable instructions. Each processing unit can be a general-purpose central processing unit (CPU), processor in an application-specific integrated circuit (ASIC) or any other type of processor. In a multi-processing system, multiple processing units execute computer-executable instructions to increase processing power. For example, FIG. 7 shows a central processing unit 710 as well as a graphics processing unit or co-processing unit 715. The tangible memory 720, 725 may be volatile memory (e.g., registers, cache, RAM), non-volatile memory (e.g., ROM, EEPROM, flash memory, etc.), or some combination of the two, accessible by the processing unit(s). The memory 720, 725 stores software 780 implementing one or more innovations described herein, in the form of computer-executable instructions suitable for execution by the processing unit(s).

A computing system may have additional features. For example, the computing environment 700 includes storage 740, one or more input devices 750, one or more output devices 760, and one or more communication connections 770. An interconnection mechanism (not shown) such as a bus, controller, or network interconnects the components of the computing environment 700. Typically, operating system software (not shown) provides an operating environment for other software executing in the computing environment 700, and coordinates activities of the components of the computing environment 700.

The tangible storage 740 may be removable or non-removable, and includes magnetic disks, magnetic tapes or cassettes, CD-ROMs, DVDs, or any other medium which can be used to store information in a non-transitory way, and which can be accessed within the computing environment 700. The storage 740 stores instructions for the software 780 implementing one or more innovations described herein.

The input device(s) 750 may be a touch input device such as a keyboard, mouse, pen, or trackball, a voice input device, a scanning device, or another device that provides input to the computing environment 700. The output device(s) 760 may be a display, printer, speaker, CD-writer, or another device that provides output from computing environment 700.

The communication connection(s) 770 enable communication over a communication medium to another computing entity. The communication medium conveys information such as computer-executable instructions, audio or video input or output, or other data in a modulated data signal. A modulated data signal is a signal that has one or more of its characteristics set or changed in such a manner as to encode information in the signal. By way of example, and not limitation, communication media can use an electrical, optical, RF, or other carrier.

Some embodiments of the disclosed methods can be performed using computer-executable instructions implementing all or a portion of the disclosed technology in a computing cloud 790. For example, the disclosed methods can be executed on processing units 710, 715 located in the computing environment 730 and/or on servers located in the computing cloud 790.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods.

Any of the disclosed methods can be implemented as computer-executable instructions stored on one or more computer-readable storage media (e.g., one or more optical media discs, volatile memory components (such as DRAM or SRAM), or non-volatile memory components (such as flash memory or hard drives)) and executed on a computer (e.g., any commercially available computer, including smart phones or other mobile devices that include computing hardware). As used herein, the term computer-readable storage media does not include communication connections, such as signals, carrier waves, or other transitory signals. Any of the computer-executable instructions for implementing the disclosed techniques as well as any data created and used during implementation of the disclosed embodiments can be stored on one or more computer-readable storage media. The computer-executable instructions can be part of, for example, a dedicated software application or a software application that is accessed or downloaded via a web browser or other software application (such as a remote computing application). Such software can be executed, for example, on a single local computer (e.g., any suitable commercially available computer) or in a network environment (e.g., via the Internet, a wide-area network, a local-area network, a client-server network (such as a cloud computing network), or other such network) using one or more network computers.

For clarity, only certain selected aspects of the software-based implementations are described. Other details that are well known in the art are omitted. For example, it should be understood that the disclosed technology is not limited to any specific computer language or program. For instance, aspects of the disclosed technology can be implemented by software written in C++, Java, Perl, any other suitable programming language. Likewise, the disclosed technology is not limited to any particular computer or type of hardware. Certain details of suitable computers and hardware are well known and need not be set forth in detail in this disclosure.

It should also be well understood that any functionality described herein can be performed, at least in part, by one or more hardware logic components, instead of software. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

Furthermore, any of the software-based embodiments (comprising, for example, computer-executable instructions for causing a computer to perform any of the disclosed methods) can be uploaded, downloaded, or remotely accessed through a suitable communication means. Such suitable communication means include, for example, the Internet, the World Wide Web, an intranet, software applications, cable (including fiber optic cable), magnetic communications, electromagnetic communications (including RF, microwave, and infrared communications), electronic communications, or other such communication means.

### VI. Examples

### Example 1

Table 3 shows an exemplary set of calculations to determine the amount of effective energy required for a ruminant animal weighing 350 kg to gain 2.5 kg of live weight if the animal consumes 9.246 kg of a diet containing 10.81 MJ/kg of EE per kg of dry weight and 93% organic matter, with 46% of dietary organic matter fermented in the rumen to produce microbial protein and short chain fatty acids in molar proportions of 65 acetate:25 propionate: 10 butyrate.

**Table 3**

| **Term** | **Value** | **Calculation** |
|---|---|---|
| Empty body weight 1 (EBW1) | 309.62 kg | (350 kg - 2.50 kg) × 0.891 |
| Empty body weight 2 (EBW2) | 311.85 kg | 350 kg × 0.891 |
| Maintenance heat (MH) | 23,302 kJ | (EBW1^0.75) × 314 kJ/1000 |
| Methane (CH₄) | 10,913 kJ | |
| Body protein for EBW1 | 58.22 kg | (-2.418 + (0.235×EBW1) - (0.00013×EBW1^2)) |
| Body protein for EBW2 | 57.88 kg | (-2.418 + (0.235×EBW2) - (0.00013×EBW2^2)) |
| Protein retention (PR) | 344 g | body protein for EBW1 - body protein for EBW2 |
| Body lipid for EBW 1 | 63.44 kg | (-0.61 + (0.037×EBW1) + (0.00054×EBW1^2) |
| Body lipid for EBW2 | 62.61 kg | (-0.61 + (0.037×EBW2) + (0.00054×EBW2^2) |
| Lipid retention (LR) | 830 g | body lipid for EBW1 - body lipid for EBW2 |
| Effective Energy Requirement | 100 MJ | (MH + CH4+ (PR × 50) + (LR × 56))/1000 |

### Example 2

An exemplary diet was prepared according to methods described and used to determine the requirement for metabolizable AA relative to EE requirement for animals of varying live body weight and having different rates of ADG. The diet was formulated to contain 14% protein and 10.81 MJ of EE per kg of dry weight was estimated to contain 93% organic matter with 46% of the organic matter fermented in the rumen causing evolution of short chain fatty acids in molar proportions of 65 acetate:25 propionate: 10 butyrate. The predicted intake of diet and EE and the amounts of maintenance energy (heat energy), methane energy and grams of protein and lipid retained by the animal are presented in Table 4. The amounts of essential amino acids and the ratio of essential metabolizable AA relative to EE (grams:MJ) to support metabolic functions and protein gain are presented in Table 4.

**Table 4**

| **Ideal AA:EE For Growing Ruminants** | | | | | | |
|---|---|---|---|---|---|---|
| **Live weight, lb** | **400** | **400** | **500** | **500** | **600** | **600** |
| Live weight, kg | 181.4 | 181.4 | 227 | 227 | 272 | 272 |
| DMI, kg/d | 3.53 | 5.45 | 4.00 | 6.13 | 4.45 | 6.71 |
| ADG, kg/d | 1.00 | 2.00 | 1.00 | 2.00 | 1.00 | 2.00 |
| EE required, MJ | 38.2 | 58.9 | 43.2 | 65.9 | 48.1 | 72.6 |
| EBW initial, kg | 148.8 | 148.8 | 188.8 | 188.8 | 228.8 | 228.8 |
| EBW final, kg | 149.6 | 150.5 | 189.7 | 190.5 | 229.7 | 230.5 |
| Protein retained, g/d | 172.7 | 345.3 | 163.6 | 326.9 | 154.4 | 308.6 |
| Lipid retained, g/d | 174.4 | 349.7 | 212.5 | 425.8 | 250.5 | 501.9 |
| Methane energy, kJ | 4167 | 6433 | 4722 | 7229 | 5256 | 7922 |
| Metabolic energy, kJ | 15579 | 15611 | 18410 | 18440 | 21102 | 21131 |

| AA requirement g/d | | | | | | |
|---|---|---|---|---|---|---|
| Met | 8.67 | 14.44 | 8.90 | 14.73 | 9.11 | 14.98 |
| Lys | 28.02 | 46.70 | 28.77 | 47.62 | 29.46 | 48.43 |
| His | 10.85 | 18.10 | 11.14 | 18.45 | 11.41 | 18.76 |
| Phe | 15.63 | 25.98 | 16.06 | 26.51 | 16.45 | 26.97 |
| Thr | 17.44 | 28.87 | 17.93 | 29.48 | 18.39 | 30.01 |
| Leu | 30.83 | 50.47 | 31.78 | 51.61 | 32.66 | 52.62 |
| Val | 18.41 | 30.13 | 18.97 | 30.82 | 19.50 | 31.42 |
| Arg | 29.06 | 48.41 | 29.83 | 49.37 | 30.55 | 50.21 |
| Ile | 13.12 | 21.45 | 13.53 | 21.94 | 13.91 | 22.37 |
| Trp | 2.22 | 3.65 | 2.28 | 3.73 | 2.34 | 3.80 |

| Ideal AA:EE ratios, g/MJ | | | | | | |
|---|---|---|---|---|---|---|
| Met | 0.227 | 0.245 | 0.206 | 0.224 | 0.189 | 0.206 |
| Lys | 0.735 | 0.793 | 0.666 | 0.723 | 0.612 | 0.667 |
| His | 0.284 | 0.307 | 0.258 | 0.280 | 0.237 | 0.258 |
| Phe | 0.410 | 0.441 | 0.372 | 0.403 | 0.342 | 0.372 |
| Thr | 0.457 | 0.490 | 0.415 | 0.448 | 0.382 | 0.413 |
| Leu | 0.808 | 0.857 | 0.735 | 0.784 | 0.679 | 0.725 |
| Val | 0.482 | 0.512 | 0.439 | 0.468 | 0.405 | 0.433 |
| Arg | 0.762 | 0.822 | 0.690 | 0.750 | 0.635 | 0.692 |
| Ile | 0.344 | 0.364 | 0.313 | 0.333 | 0.289 | 0.308 |
| Trp | 0.058 | 0.062 | 0.053 | 0.057 | 0.049 | 0.052 |
| | | | | | | |

| **Live weight, lb** | **700** | **700** | **800** | **800** | **1200** | **1200** |
|---|---|---|---|---|---|---|
| Live weight, kg | 318 | 318 | 362 | 362 | 544 | 544 |
| DMI, kg/d | 4.89 | 7.38 | 5.33 | 7.94 | 6.98 | 10.3 |
| ADG, kg/d | 1.00 | 2.00 | 1.00 | 2.00 | 1.00 | 2.00 |
| EE required, MJ | 52.9 | 79.30 | 57.6 | 85.8 | 75.5 | 11.3 |
| EBW initial, kg | 268.8 | 268.80 | 308.8 | 308.8 | 468.9 | 468.9 |
| EBW final, kg | 269.7 | 270.60 | 309.7 | 310.6 | 469.8 | 470.6 |
| Protein retained, g/d | 145.3 | 290.3 | 136.1 | 272.0 | 99.5 | 198.7 |
| Lipid retained, g/d | 288.6 | 577.9 | 326.6 | 654.0 | 478.8 | 958.4 |
| Methane energy, kJ | 5775 | 8655 | 6286 | 9368 | 8243 | 12157 |
| Metabolic energy, kJ | 23684 | 23711 | 26174 | 26201 | 35465 | 35489 |

| AA requirement g/d | | | | | | |
|---|---|---|---|---|---|---|
| Met | 9.31 | 15.24 | 9.50 | 15.47 | 10.21 | 16.35 |
| Lys | 30.11 | 49.28 | 30.73 | 50.01 | 33.02 | 52.86 |
| His | 11.66 | 19.09 | 11.90 | 19.37 | 12.78 | 20.47 |
| Phe | 16.83 | 27.45 | 17.19 | 27.87 | 18.50 | 29.49 |
| Thr | 18.82 | 30.56 | 19.24 | 31.05 | 20.76 | 32.91 |
| Leu | 33.49 | 53.65 | 34.28 | 54.57 | 37.17 | 58.04 |
| Val | 20.00 | 32.03 | 20.47 | 32.58 | 22.19 | 34.66 |
| Arg | 31.23 | 51.09 | 31.88 | 51.86 | 34.26 | 54.82 |
| Ile | 14.27 | 22.82 | 14.61 | 23.21 | 15.86 | 24.70 |
| Trp | 2.40 | 3.88 | 2.46 | 3.94 | 2.66 | 4.19 |

| Ideal AA:EE ratios, g/MJ | | | | | | |
|---|---|---|---|---|---|---|
| Met | 0.176 | 0.192 | 0.165 | 0.180 | 0.135 | 0.147 |
| Lys | 0.569 | 0.621 | 0.534 | 0.583 | 0.437 | 0.475 |
| His | 0.220 | 0.241 | 0.207 | 0.226 | 0.169 | 0.184 |
| Phe | 0.318 | 0.346 | 0.299 | 0.325 | 0.245 | 0.265 |
| Thr | 0.356 | 0.385 | 0.334 | 0.362 | 0.275 | 0.296 |
| Leu | 0.633 | 0.677 | 0.596 | 0.636 | 0.492 | 0.522 |
| Val | 0.378 | 0.404 | 0.356 | 0.380 | 0.294 | 0.312 |
| Arg | 0.591 | 0.644 | 0.554 | 0.604 | 0.454 | 0.493 |
| Ile | 0.270 | 0.288 | 0.254 | 0.270 | 0.210 | 0.222 |
| Trp | 0.045 | 0.049 | 0.043 | 0.046 | 0.035 | 0.038 |

### Example 3

Table 5 shows an exemplary set of calculations to determine the amount of metabolizable amino acids supplied by rumen micro-organisms to support productive function of a lactating ruminant, i.e., lactating Holstein cow. A diet comprised of forages and grains was formulated to provide 1.68 Mcal (7.03 MJ) of NE and fed at 25.11 kg/d, to a non-pregnant animal weighing 590 kg gaining .11 kg/d and producing 45.36 kg/d of milk (4.90% lactose, 3.7% fat, 3.30% protein). The nutrient content and nutrient digestibility in the rumen (kg, in parenthesis) were as follows: 17.9% CP (1.317 kg), 39.4% NFC (4.305 kg), 29.3% NDF (2,970 kg). The SD for protein and NSC was assumed to be 6% and the SD for fiber was set at 3%, with ruminal bacteria fractionated into respective pools as follows: SA = 80% and LA = 20%. The LD was assumed to = 10%. The MTP was assumed to be 85% of microbial crude protein and it was further assumed that 85% of true protein was absorbed from the intestines (MTPD). The calculations show the amount of microbial protein synthesized per day and the amounts of metabolizable microbial amino acids absorbed from the small intestine. Supply of individual essential AA was calculated by multiplying the percentage of each AA in microbial true protein by the flow of microbial true protein.

**Table 5**

| **Determination of Metabolizable Microbial Amino Acid Supply** | | |
|---|---|---|
| **Term** | **Value** | **Calculation** |
| Efficiency of starch fermenting micro-organisms | 23.9 g/kg | MOEFF_{starch} = (7.1 + (3.416 x DR) - (965.3 x DR²) x SA) + (7.1 + (3.416 x DR) - (965.3 x DR²) x LA) |
| Efficiency of NDF fermenting micro-organisms | 19.5 g/kg | MOEFF_{NDF} = (1.7 + (368.7 x DR) - (586.9 x DR²) x SA) + (1.7 + (368.7 x DR) - (586.9 x DR²) x LA) |
| Efficiency of protein fermenting micro-organisms | 43.0 g/kg | MOEFF_{Pr} = (9.3 + (599.2 x DR) - (1445.6 x DR²) x SA) + (9.3 + (599.2 x DR) - (1445.6 x DR²) x LA) |
| Metabolizable Microbial Protein (MMP) Synthesized | 1,006 g | Microbial Protein = (MOEFF_{starch} x kg starch fermented) + (MOEFF_{NDF} x kg NDF fermented) + (MOEFF_{Pr} x kg protein fermented) x MTP x MTPD |
| Met | 27.2 g | MMP x 2.7%/100 |
| Lys | 82.5 g | MMP x 8.2%/100 |
| His | 27.2 g | MMP x 2.7%/100 |
| Phe | 52.3 g | MMP x 5.2%/100 |
| Thr | 56.3 g | MMP x 5.6/100 |
| Leu | 75.4 g | MMP x 7.5%/100 |
| Val | 62.4 g | MMP x 6.2%/100 |
| Arg | 70.4 g | MMP x 7.0%/100 |
| Ile | 59.1 g | MMP x 5.88%/100 |
| Trp | 16.4 g | MMP x 1.63%/100 |

### Example 4

Table 6 shows exemplary AA:ME ratios required to support productive function of a lactating ruminant, i.e., lactating Holstein cow secreting varying amounts of milk with low, moderate, or high percentages of protein. An archetypical diet comprised of forages and grains was formulated to contain 10.9 MJ of ME/kg, 17.9% CP, 39.4% NFC, and 29.3% NDF. A milking Holstein cow (non-pregnant), weighing 590 kg and gaining .11 kg/d producing was used to model adjustments required to optimize the composition of the diet. The secretion of milk per day and the percentage of protein in secreted milk was varied within biological limits typical for a cow of this breed and weight. The amount of the diet consumed per day was adjusted to meet the ME requirement for body weight maintenance, gain, and milk secretion. The diet was further adjusted to provide essential AA (g/d) to meet or exceed requirements for EAA. The ideal AA:ME ratios (grams of AA:MJ) are presented in the table. The EAA (g/d) supply was estimated as the sum of microbial AA synthesized in the rumen and RUP and encapsulated AA provided by the adjusted diet.

**Table 6**

| **Ideal AA:ME ratios for lactating ruminants** | | | | | | | | | |
|---|---|---|---|---|---|---|---|---|---|
| **Milk, kg/d** | 36.3 | 36.3 | 36.3 | 45.4 | 45.4 | 45.4 | 54.4 | 54.4 | 54.4 |
| **Milk fat, %** | 3.70 | 3.70 | 3.70 | 3.70 | 3.70 | 3.70 | 3.70 | 3.70 | 3.70 |
| **Milk protein, %** | 3.30 | 3.10 | 2.90 | 3.30 | 3.10 | 2.90 | 3.30 | 3.10 | 2.90 |
| **Milk lactose, %** | 4.90 | 4.90 | 4.90 | 4.90 | 4.90 | 4.90 | 4.90 | 4.90 | 4.90 |
| **Feed intake, kg/d** | 21.08 | 20.86 | 20.62 | 24.95 | 29.18 | 24.36 | 28.79 | 28.36 | 27.98 |
| **ME intake MJ/d** | 228 | 226 | 223 | 270 | 267 | 264 | 312 | 308 | 303 |
| **Ideal AA:ME ratio, g/MJ** | | | | | | | | | |
| **Met** | 0.2511 | 0.2414 | 0.2316 | 0.2603 | 0.2503 | 0.2399 | 0.2672 | 0.2568 | 0.2469 |
| **Lys** | 0.8836 | 0.8485 | 0.8161 | 0.9175 | 0.8814 | 0.8438 | 0.9426 | 0.9052 | 0.8694 |
| **His** | 0.3277 | 0.3148 | 0.3080 | 0.3400 | 0.3268 | 0.3130 | 0.3491 | 0.3354 | 0.3223 |
| **Phe** | 0.4681 | 0.4498 | 0.4313 | 0.4855 | 0.4666 | 0.4470 | 0.4983 | 0.4788 | 0.4601 |
| **Thr** | 0.4332 | 0.4174 | 0.4013 | 0.4474 | 0.4310 | 0.4140 | 0.4579 | 0.4409 | 0.4247 |
| **Leu** | 0.9353 | 0.5988 | 0.8619 | 0.9689 | 0.9313 | 0.8922 | 0.9939 | 0.9549 | 0.9176 |
| **Val** | 0.5261 | 0.5061 | 0.4857 | 0.5445 | 0.5237 | 0.5021 | 0.5580 | 0.5365 | 0.5159 |
| **Arg** | 0.5074 | 0.4917 | 0.4758 | 0.5194 | 0.5032 | 0.4863 | 0.5283 | 0.5115 | 0.4954 |
| **Ile** | 0.4372 | 0.4197 | 0.4020 | 0.4537 | 0.4357 | 0.4169 | 0.4659 | 0.4472 | 0.4293 |
| **Trp** | 0.1238 | 0.1183 | 0.1128 | 0.1295 | 0.1238 | 0.1180 | 0.1336 | 0.1278 | 0.1222 |

### Example 5

A feeding study was conducted to evaluate growth and carcass characteristics of growing ruminant animals fed adjusted diets. Beef cattle (n = 1,344, avg initial weight of 850 lb) in a commercial feedlot were blocked by weight and randomly assigned to one of 16 pens with approximately 84 animals assigned per pen. The unadjusted (control) or the adjusted diet was fed to eight pens of cattle. Cattle were fed twice daily for ad libitum feed consumption. The experiment was conducted over a 142-day period. Animals were weighed at an interim period of 58 days on feed.

The amino acid requirement of the animals was calculated as described above, with amino acid requirements for maintenance and tissue growth calculated and summed. The predicted supply of amino acids to the small intestine was calculated by summing amino acid flow from rumen microbes and predicted dietary amino acid flow as outlined above. The total quantity of amino acids flowing to the small intestine then was multiplied by 0.85 assuming an intestinal absorption efficiency of 85%. The supply of absorbed (metabolizable) amino acids was expressed as a percentage of estimated amino acid requirement. For the adjusted diet, distillers grains was increased and AjiPro^{®}-L (rumen protected lysine supplement, Ajinomoto Animal Nutrition North America, Inc., Chicago, IL) was added to increase estimated amino acid supply relative to dietary EE. The diet formulations are shown in Table 7.

**Table 7**

| **Ingredient and Chemical Composition of Diets** | | |
|---|---|---|
| **Ingredient, % of dry weight** | **Unadjusted** | **Adjusted** |
| Corn earlage (cobbage) | 29.1 | 29.2 |
| Wheat - soft white winter | 35.2 | 28.0 |
| Corn grain, dry-rolled | 16.9 | 12.7 |
| Corn distillers syrup | 5.0 | 5.0 |
| BVF finisher supplement | 3.3 | 3.3 |
| Corn distillers grain | 3.3 | 14.4 |
| Alfalfa hay | 3.5 | 3.5 |
| Fat, tallow | 3.7 | 3.7 |
| AjiPro^{®} encapsulated lysine | 0.0 | 0.14 |
| Total | 100.0 | 100.0 |

| **Chemical composition** | | |
|---|---|---|
| Moisture, % | 26.2 | 27.8 |
| Dry matter, % | 73.8 | 72.2 |
| Crude protein, % of dry matter (DM) | 13.0 | 15.5 |
| MP Arg, % of requirement (1^{st} period) | 93 | 100 |
| MP Lys, % of requirement (1^{st} period) | 90 | 99 |
| MP Met, % of requirement (1^{st} period) | 102 | 109 |
| NPN, % of DM | 1.9 | 2.0 |
| ADF, % of DM | 8.4 | 7.7 |
| NDF, % of DM | 13.8 | 15.1 |
| EE intake, MJ/d | | 102 |
| NEm, mcal/lb | 0.89 | 0.90 |
| Neg, mcal/lb | 0.57 | 0.57 |
| Digestible energy, mcal/lb | 1.56 | 1.57 |
| Metabolizable energy, mcal/lb | 1.28 | 1.28 |
| Effective energy ratio (first period) | 1.24 | 1.19 |

The performance data were separated into two periods: day 1 to day 58 and day 1 to finish. As shown in Table 8, during the first 58 days, animals fed the adjusted diet had an ADG of 4.62 lbs. which was 7% greater than animals fed the unadjusted diet. Animals fed the adjusted diet further showed a 7% improvement in feed efficiency compared to animals fed the unadjusted diet. Dry matter intake did not differ between treatments. The adjusted diet was more costly per unit of dry weight; however, the cost of gain was lower for animals fed the adjusted diet because daily gain and feed efficiency both were improved by feeding the adjusted diet. Improved ADG and feed efficiency were statistically significant.

**Table 8**

| **Initial Performance of Cattle (Days 1-58)** | | | | | |
|---|---|---|---|---|---|
| **Item** | **Unadjusted Diet** | **Adjusted Diet** | **SEM** | **Treatment P-Value** | **Block P-Value** |
| Initial wt, lb | 851 | 847 | 19.2 | 0.87 | 0.64 |
| Day 58 wt, lb | 1103 | 1118 | 11.6 | 0.36 | 0.06 |
| ADG, lb | 4.32 | 4.62 | 0.086 | 0.03 | 0.37 |
| Feed:Gain | 4.83 | 4.48 | 0.075 | 0.01 | 0.18 |
| DMI, lb/d | 20.8 | 20.7 | 0.18 | 0.59 | 0.01 |
| Feed Cost/hd, $/hd | 118.47 | 125.04 | 12.54 | 0.72 | 0.14 |
| Cost of Gain, $/CWT | 57.11 | 56.64 | 0.94 | 0.73 | 0.19 |

Overall performance is presented in Table 9 and carcass measurements are presented in Table 10. Over the entire 142-day study, animals fed the adjusted diet had an improved feed efficiency of 2.5% and ADG was numerically improved. Yield of carcass, based on 4 % shrink of final live weight, tended P<0.18) to be greater for animals fed the adjusted vs. unadjusted diet (904 lbs vs. 892 lbs). Marbling score was greater for animals fed the unadjusted diet, but animals fed the adjusted diet averaged 5 % prime carcasses compared to 1% prime for animals fed the unadjusted diet. Subcutaneous fat and yield grade were lower and ribeye area was greater for animals fed the adjusted diet.

**Table 9**

| **Overall Performance of Cattle (Days 1-142)** | | | | | |
|---|---|---|---|---|---|
| **Item** | **Unadjusted Diet** | **Adjusted Diet** | **SEM** | **Treatment P-Value** | **Block P-Value** |
| Initial wt, lb | 851 | 847 | 19.2 | 0.87 | 0.64 |
| Final live wt (4% shrink), lbs | 1416 | 1425 | 7.6 | 0.42 | 0.06 |
| Final wt, 63.3% adjusted, lbs | 1417 | 1436 | 6.6 | 0.06 | 0.04 |
| 4% shrunk ADG, lb | 3.97 | 4.07 | 0.06 | 0.24 | 0.47 |
| Carcass adjusted ADG, lb | 3.97 | 4.17 | 0.04 | 0.01 | 0.35 |
| DMI, lb/d | 22.4 | 22.3 | 0.24 | 0.95 | 0.88 |
| F/G 4% shrunk wt | 5.63 | 5.49 | 0.03 | 0.01 | 0.03 |
| F/G carcass adjusted | 5.63 | 5.39 | 0.04 | <0.01 | 0.20 |
| Feed cost, $/hd | 310.59 | 312.76 | 8.20 | 0.85 | 0.35 |
| Cost of gain, $/CWT Carc adj | 55.01 | 53.18 | 0.44 | 0.01 | 0.20 |
| Cost of gain, $/CWT 4% shrunk | 55.04 | 54.12 | 0.34 | 0.07 | 0.03 |

It is concluded that animals fed the adjusted diet exhibited improved energy efficiency because metabolizable amino acids provided by the adjusted diet were more closely aligned with amino acid requirement and with the EE content of the diet.

Using feed cost and initial animal purchase price to compute net difference, animals fed the treatment diet had a $17.54 greater profit per animal than animals fed the control diet (Table 10). This equates to a 4.8 % improvement in profit per animal.

**Table 10**

| **Carcass Characteristics** | | | | | |
|---|---|---|---|---|---|
| **Item** | **Unadjusted Diet** | **Adjusted Diet** | **SEM** | **Treatment P-Value** | **Block P-Value** |
| Hot carcass wt, lbs | 892 | 904 | 4.2 | 0.06 | 0.04 |
| Marbling score | 507 | 474 | 8.1 | 0.01 | 0.02 |
| Yield grade | 2.34 | 2.19 | 0.04 | 0.02 | <0.01 |
| 12th rib fat, in | 0.65 | 0.62 | 0.01 | 0.04 | 0.04 |
| REA, in² | 14.41 | 14.91 | 0.07 | <0.01 | <0.01 |
| Prime, % | 1.68 | 5.65 | 3.78 | 0.47 | 0.66 |
| Choice, % | 89.23 | 84.67 | 7.17 | 0.66 | 0.30 |
| Select, % | 9.16 | 8.35 | 5.90 | 0.92 | 0.24 |
| No Roll, % | 0.08 | 1.33 | 1.02 | 0.34 | 0.47 |
| Yield Grade 1, % | 15.1 | 13.7 | 3.57 | 0.78 | 0.1 |
| Yield Grade 2, % | 47.6 | 48.7 | 2.53 | 0.77 | 0.63 |
| Yield Grade 3, % | 33.1 | 34.4 | 3.14 | 0.77 | 0.0791 |
| Yield Grade 4, % | 4.0 | 3.3 | 0.88 | 0.59 | 0.13 |
| Yield Grade 5, % | 0.28 | 0.02 | 0.13 | 0.12 | 0.14 |
| Carcass value, $/hd | 1,760.54 | 1,774.34 | 15.4 | 0.54 | 0.25 |
| Agribeef Grid, $/CWT: Base Price $197.74; Choice/select spread $11.00; Prime premium $10.50; No roll discount ($10.00); YG 1 $3.50; YG 2 $2.00; YG 4 ($15.00); YG 5 ($20.00); Overweight (>1,050 lb) ($25.00) | | | | | |

Collectively these results demonstrate the potential benefits when an adjusted diet is fed to growing ruminant animals.

### Example 6

A feeding study was conducted with growing ruminant animals to evaluate the performance and health of animals fed adjusted diets formulated with no NDF provided from a forage fiber source ('no roughage diet').

The hypothesis tested in this study was that adjusting diets to meet the AA:EE requirement and further adjusting the diet to meet the peptide-nitrogen and ammonia requirement of rumen microflora would prevents overconsumption of fermentable starch, thereby improving animal performance and value of the edible meat produced. Unadjusted diets typically fed to growing ruminants contain high starch levels and have AA:EE ratios that are inadequate to meet the animal's requirement. The imbalance in AA:EE results in the animal overconsuming energy and more specifically, overconsuming rumen fermentable starch, in an attempt to consume sufficient amino acids to support lean tissue gain. The overconsumption of fermentable starch results in large amounts of lactic acid being produced, which causes metabolic deraignment and acidosis of the animal. As a preventative of metabolic deraignment, unadjusted diets containing high amounts of fermentable starch are formulated to contain some amount of less fermentable carbohydrate. Typically, 6 to 10% of the diet dry matter is comprised of a forage-derived fiber source. This level of forage dilutes starch intake, increases mixing of rumen contents and increases rumination (buffering) activity of the animal. A surprising discovery is that adjusted diets having optimized AA:EE ratios can ameliorate overconsumption of fermentable starch thereby alleviating the need for forage fiber in adjusted diets.

To test the efficacy of removing forage fiber from high starch (corn) diets, growing ruminant animals were placed in outdoor pens (approximately 70 head per pen, two pens) and fed a highly fermentable corn-based diets (76% corn on a dry weight basis). The diet was adjusted to provide the ideal ratio of AA:EE according to procedures detailed previously. The adjusted diet that was fed is presented in Table 11.

**Table 11**

| **Composition of Diet Fed to Growing Ruminants** | |
|---|---|
| **Item** | |
| Ingredient, % of dry weight | |
| Whole corn grain | 76.42 |
| Soybean meal | 5.86 |
| Distillers dried grains | 11.42 |
| Cottonseed meal | 2.71 |
| Urea | .66 |
| Minerals, vitamins, additives | 2.93 |
| Total | 100.00 |
| Diet Dry matter, % | 90.1 |
| Diet Organic matter, % of dry matter | 93.7 |
| Diet Protein, % of dry matter | 15.68 |
| Diet NDF, % of dry matter | 14.26 |
| Diet Lipid, % of dry matter | 4.45 |
| Predicted as fed intake, lb/day | 24.0 |
| Feed:gain ratio | 5.69 |
| EE ratio | 1.05 |
| RD peptide ratio | 1.04 |
| RD ammonia ratio | .93 |

| Metabolizable AA, % of requirement | |
|---|---|
| Arg | 120 |
| Lys | 109 |
| Met | 121 |
| His | 143 |
| Thr | 147 |
| Phe | 189 |
| Leu | 185 |
| Val | 178 |
| Ile | 205 |
| Trp | 374 |

Animals weighed approximately 600 lb when the study was initiated and were fed the adjusted diet for more than 200 days, achieving a final body weight of approximately 1300 lb. At the conclusion of the feeding period, the animals were harvested at a commercial facility and carcass data was measured.

Negative consequences of the no forage fiber diet would have been evidenced from poor carcass quality grade and yield grade, particularly evident when compared to the average values for quality and yield for the commercial harvesting facility which would have been based upon cattle fed typical commercial diets.

The percentage of prime carcass was measured at 18.9 % compared to an average of 2.4% prime for other animals being processed at the harvesting facility. Furthermore, animals fed the adjusted diet had increased choice or greater carcasses compared to the plant average (95 .8% compared to 71.7%). The results of this study showed that adjusting diets to meet AA:EE requirements increased prime carcasses almost 8-fold and choice or higher quality grade 34%. Yield grades were higher for calves fed diets balanced for AA:EE compared to plant average, but distribution was not substantially different. Animals fed the adjusted diet netted a $49 per head premium compared to the plant average for animals fed typical unadjusted diets. Adjusting diets to meet an AA:EE ratio improved quality grade of the carcass and netted additional revenue because of better meat quality. Furthermore, a surprising observation was that no adverse health effects were noted when animals were fed the highly fermentable diet devoid of forage-fiber. This was attributed to the adjusted diet having AA:EE ratios that attenuated overconsumption of the diet. The results of this study demonstrated benefits for feeding adjusted diets to growing cattle as a means of improving meat quality, maintaining animal health, and alleviating the need for forage-fiber in the diet.

**Table 12**

| **Carcass Measurements** | | |
|---|---|---|
| **Carcass Measurement** | **Plant Average Carcass Value, %** | **Measured Carcass Value, %** |
| Prime Quality Grade | 2.39 | 18.87 |
| Choice or Higher Quality Grade | 71.69 | 95.82 |
| Yield Grade 1 | 15.49 | 3.39 |
| Yield Grade 2 | 36.98 | 25.98 |
| Yield Grade 3 | 33.22 | 48.23 |
| Yield Grade 4 | 12.24 | 22.40 |

### Example 7

Lactating cows were fed an unadjusted or an adjusted diet in a study designed to measure feed intake and milk production. The cows weighed 1,540 lb when the study was initiated and were gaining an average of 0.10 lb of body weight per day. Evaluation of the unadjusted diet showed that cows were overconsuming energy because the unadjusted diet had a lysine deficiency and was marginal in histidine. An adjusted diet was prepared by adding bloodmeal and AjiPro^{®}-L supplement t to the unadjusted diet such that the adjusted diet provided additional metabolizable amino acids to match the predicted intake of ME. The diets are shown in Table 13.

**Table 13**

| **Diets Fed to Lactating Dairy Cows** | | |
|---|---|---|
| **Component, % as fed** | **Unadjusted diet** | **Adjusted diet** |
| corn silage | 40.0 | 40.0 |
| alfalfa haylage | 17.8 | 17.8 |
| alfalfa hay | 4.4 | 4.4 |
| corn grain | 15.5 | 14.6 |
| brewers grain | 12.9 | 12.9 |
| soybean hulls | 2.6 | 2.6 |
| soybean meal | 1.4 | 1.4 |
| AminoPlus^{®} protein* | 3.3 | 3.3 |
| Mineral mix | 1.6 | 2.0 |
| Bloodmeal | 0.5 | 0.8 |
| AjiPro^{®}-L supplement | | 0.15 |
| Total | 100.0 | 100.0 |
| Dry matter, % | 48.9 | 49.1 |
| Organic matter, % of dry matter | 91.1 | 91.1 |
| Protein, % of dry matter | 16.1 | 16.6 |
| NDF, % of dry matter | 28.5 | 28.3 |
| Lipid, % of dry matter | 4.9 | 4.9 |
| MP AA supplied, g/d | | |
| Lys | 181 | 197 |
| Met | 73 | 73 |
| His | 75 | 80 |
| MP Met:ME ratio | 1.1 | 1.1 |
| MP Lys:ME ratio | 2.6 | 2.9 |
| MP His:ME ratio | 1.1 | 1.2 |

| | | |
|---|---|---|
| *Rumen-bypass soybean product (Ag Processing, Inc., Omaha, NE) | | |

As shown in Table 14, milk production per cow improved by 5 lbs/day when the adjusted diet was fed, and the milk was $.025/lb more valuable because of improved composition and quality. Net return increased by more than $1 per cow per day when cows were fed the adjusted diet.

**Table 14**

| **Milk Production By Lactating Cows** | | |
|---|---|---|
| | Unadjusted Diet | Adjusted Diet |
| Intake, lbs as fed | 131 | 129 |
| Milk, lbs | 92 | 97 |
| % Fat | 3.7 | 3.6 |
| % Protein | 2.9 | 3.0 |
| % Lactose | 4.8 | 4.9 |
| Somatic Cell Count | 109 | 46 |
| Milk value, $/lb | 2.875 | 2.899 |
| Milk value/cow, $ per day | 26.45 | 28.12 |

### Example 8

A trial was conducted to examine the benefits of feeding an adjusted diet to dairy cows. An unadjusted diet ("basal diet", "control") was prepared using a commercially available formulation program (Agricultural Modeling and Training Systems (AMTS)). The basal diet was adjusted to balance for AA content relative to metabolizable energy ("optimized diet", "treated") using the disclosed technique. The trial was conducted for 56 days on a commercial dairy farm in Michigan using Holstein and Jersey cows (n ≅ 180). The cows were divided into two equal groups and were balanced based upon breed, parity, stage of lactation (DIM), mean milk production and milk components measured one week before the co-variant adjustment period. The number of cows less than 80 DIM was equal among groups.

Cows were housed in a free stall facility and fed two separate rations. Cows were milked twice per day in a milking parlor and all milk yields were recorded. A three-week, co-variant adjustment period was followed by the 56-day test. The basal diet (control cows) contained corn silage, alfalfa haylage, corn earlage, vitamins, and minerals. The basal diet and the optimized diet (treated cows) were formulated to be iso-caloric, iso-nitrogenous and similar in ruminally degraded protein (RDP). Diets were formulated to contain a minimum of 16.6% crude protein (CP) and 6.8% rumen undegraded protein (RUP) on a DM basis. The primary sources of RUP in the basal diet were blood meal and a specially-processed soybean meal (AminoPlus^{®}; Ag Processing, Inc., Omaha, NE). The composition ingredients and the estimated nutrient composition of diets is shown in Table 15.

Rations were offered as a total mixed ration (TMR) once daily for ad libitum intake. Delivered diets were pushed up 8-10 times daily to encourage ad libitum dry matter intake (DMI). The amount of feed offered were adjusted to allow for about five percent feed refusals. Feed refusals were measured and recorded daily. The number of animals in each group and average daily milk production were reported on a daily basis to correlate with the pen mean for daily DMI. The TMR were mixed and delivered with a Lucknow feed mixer (Lucknow Products, Ontario, Canada). Feed DM was monitored daily and adjustments in amounts of feed offered were made accordingly. Feed offered and estimated consumption (offered-refusal) were recorded daily.

**Table 15**

| **Composition of Diets Fed To Lactating Dairy Cows** | | |
|---|---|---|
| **Ingredients (Lbs AF)** | **Control** | **Treatment** |
| Corn Earlage | 15.76 | 14.92 |
| Haylage | 20.55 | 19.04 |
| Corn Silage | 51.10 | 66.28 |
| Ground Corn | 3.41 | 4.83 |
| Canola Meal | 5.00 | 5.11 |
| Corn Gluten Feed | 5.06 | 2.63 |
| Wheat Straw | 1.44 | 1.30 |
| AminoPlus | 2.84 | 4.43 |
| Calcium Carbonate | 0.927 | 0.930 |
| SQ 810 Carb | 0.704 | 0.702 |
| Bloodmeal - Porcine | 0.556 | 0.557 |
| Megalac | 0.515 | 0.511 |
| Dry Distillers | 0.556 | 0.000 |
| White Salt | 0.201 | 0.201 |
| CN10 VTM | 0.102 | 0.102 |
| Urea | 0.101 | 0.164 |
| Mag Ox 56% | 0.100 | 0.100 |
| NitroShure | 0.101 | 0.000 |
| Syn Tech RGI | 0.027 | 0.027 |
| Rumensin 90 GM/LB | 0.004 | 0.004 |
| AjiPro L v3 | | 0.267 |
| Smartamine M | | 0.035 |
| **Total** | 109.04 | 122.15 |

| **Nutrients (DM basis)** | | **Control** | **Treatment** |
|---|---|---|---|
| Dry Matter | lbs | 59.00 | 59.03 |
| Dry Matter | % | 54.11 | 53.94 |
| Protein | % | 16.42 | 16.66 |
| Fat | % | 4.01 | 4.11 |
| ADF | % | 19.95 | 19.38 |
| aNDFom | % | 31.31 | 30.81 |
| peNDF | % | 22.39 | 22.44 |
| Rumen Undegrabable Protein (RUP) | % DM | 7.19 | 7.61 |
| Soluble Protein (SOL) | % DM | 6.73 | 6.85 |
| Rumen Degradable Protein (RDP) | % DM | 9.22 | 9.05 |
| RUP | % Prot. | 43.84 | 45.66 |
| SOL | % Prot. | 41.00 | 41.13 |
| RDP | % Prot. | 56.16 | 54.34 |
| Non Fiber Carbohydrate | % | 40.30 | 40.50 |
| Net Energy: lactation (ANI) | Mcal/cwt | 72.00 | 73.00 |
| Net Energy: gain | Mcal/cwt | 44.00 | 45.00 |
| Calcium | % | 1.07 | 1.05 |
| Phosphorus | % | 0.44 | 0.40 |
| Chloride | % | 0.42 | 0.46 |
| Magnesium | % | 0.33 | 0.32 |
| Potassium | % | 1.29 | 1.28 |
| Salt | % | 0.33 | 0.34 |
| Sodium | % | 0.56 | 0.54 |
| Sulfur | % | 0.22 | 0.21 |
| Cobalt | ppm | 0.39 | 0.38 |
| Copper | ppm | 15.69 | 14.84 |
| Iodine | ppm | 0.74 | 0.73 |
| Iron | ppm | 190.36 | 178.85 |
| Manganese | ppm | 87.30 | 84.87 |
| Selenium | ppm | 0.38 | 0.37 |
| Zinc | ppm | 86.83 | 85.85 |
| Vit A | KIU/lb | 2.50 | 2.40 |
| Vit D | KIU/lb | 0.70 | 0.70 |
| Vit E | IU/lb | 13.30 | 13.20 |
| Lysine - MP | grams | 185.20 | 222.80 |
| Methionine - MP | grams | 63.70 | 75.90 |
| Histidine - MP | grams | 79.70 | 81.80 |
| RUP-Lys - MP | grams | 89.10 | 141.80 |
| RUP-Met - MP | grams | 32.70 | 44.30 |
| RUP-His - MP | grams | 52.40 | 53.70 |
| RUP-LYS/MET | - | 2.73 | 3.20 |

Feed samples were collected weekly and dry matters determined and used to make adjustment in the rations (as-fed basis). Samples of individual feeds collected at the beginning were analyzed for nutrient analysis. Samples of diets were collected weekly and a composite sample formed for nutrient analysis to verify the nutrient profile of the diets consumed. Individual milk weights were automatically recorded at each milking for all animals. Milk yield was recorded for three weeks prior to the test through one week after completion to monitor the effect of transitions around the experimental diets. Mean individual milk yield was calculated for each week of the test and used in statistical analysis. Milk samples were collected from two consecutive milkings on days -7, 0, 28 and 56. Milk protein, fat, lactose, somatic cell counts, and milk urea nitrogen was determined.

Data was analyzed using a repeated measures, completely randomized design. Yields of milk and milk components were co-variant adjusted using data collected in the pre-test period. The statistical model included DIM to account for the effects of lactation stage. Significance was declared at P<0.10 unless otherwise stated.

The results of the study are presented in Table 16. Calculated pen feed intake data confirm that cows consumed similar amounts of dry matter (e.g., 62.59 DM/cow/day for the control cows versus 62.542 DM/cow/day for the treatment cows). Feeding the adjusted diet resulted in a 4.6 lb/day milk increase in milk yield versus feeding the control diet (89.7 vs 85.0 lb/day; P < .01). Feeding the adjusted diet also resulted in an increase in the percentage of protein in milk (3.22 vs. 3.06; P<.01) and a numerical increase in the percentage of fat in milk (3.75 vs. 3.66%; P = .53). The results of this study demonstrated the benefits for milk yield and milk protein percentage resulting from feeding an adjusted diet on a commercial dairy farm. The overall cost advantage of feeding the adjusted diet was estimated to be $.24/hd/day not including the benefit for milk fat increase; including the benefits associated with milk fat increase resulted in a net benefit of $1.10/hd/day.

**Table 16**

| **Milk production and composition data of dairy cows** | | | | | |
|---|---|---|---|---|---|
| ITEM | DIET | | SEM | *P VALUE* | |
| | Control | Adjusted | | Trt | Trt x Period |
| DM intake, lb/d | 62.6 | 62.5 | | | |
| Milk, lbs/d | 85.02 | 89.65 | 0.69 | <0.01 | 0.75 |
| Protein, % | 3.06 | 3.22 | 0.033 | <0.01 | <0.01 |
| Fat, % | 3.66 | 3.75 | 0.109 | 0.53 | <0.01 |
| Lactose % | 4.71 | 4.74 | 0.024 | 0.40 | 0.45 |
| Milk solids, % | 5.62 | 5.65 | 0.024 | 0.40 | 0.45 |
| MUN, mg/dL | 13.9 | 15.5 | 0.2 | <0.01 | 0.01 |

### Example 9

A continuous culture study was conducted to evaluate short chain fatty acid evolution and methane gas production when the fiber content was reduced in adjusted diets and when the culture system was fed lesser amounts of the adjusted diet. The continuous culture system was used in a manner emulating fermentation that would occur in the rumen of a ruminant animal, thereby providing results that are relevant to the feeding of ruminant animals.

A ruminally cannulated beef cow fed an unadjusted diet composed of a grain mix (15-20 lb/ day) and hay (6 lb/day) was used as a source of rumen inoculum. Four continuous culture fermentation systems, as described by Teather and Sauer (1988) were used in a 4x3 incomplete Latin square design, with 4 treatments and 3 periods of 10 days each (7 days adaptation followed by 3 days of sample collection).

Whole ruminal contents (liquid and particle matter) were collected 2-4 hours after the morning feeding, strained using a double-layered cheesecloth and then transferred into the lab in insolated sealed 3.5 L jar. The rumen material was collected on the first day of each period and added to the fermenters within 30 min of the collection. Approximately 650 ml of the ruminal fluid was added to each of four the fermenters, containing 100 ml of prewarmed buffer (Weller and Pilgrim, 1974). Anaerobic conditions were maintained by infusing CO₂ at 45 ml/min. Cultures were stirred continuously at 35 rpm and fermenter pH was measured daily before addition of feed using a portable pH meter at 0800, 1600 and 2300 hr. Fermenter pH was maintained around 6.0 (±0.1) by adjusting buffer pH level. Fermenter temperature was maintained at 39°C using a circulating water bath. Buffer was delivered continuously at a flow rate (8%/h liquid dilution rate), using a precision pump.

An unadjusted or adjusted diet was provided to the fermenter cultures to evaluate the effects of diet adjusted on fermentation characteristics, including methane gas production. The unadjusted diet was evaluated and adjusted to optimize the provision of RDN and peptide N, and AA relative to estimated EE. The composition of the first adjusted diet was further modified by eliminating the forage fiber component. The adjusted diet having forage fiber or not having forage fiber was then provided to the continuous fermenter systems in amounts matching the amount provided when the unadjusted diet was provided, or at an amount equal to 80% of the amount provided by the unadjusted diet. The nutrient composition of the diets provided to the fermenters is presented by Table 17.

**Table 17**

| Composition of Diets Provided to Fermenters | | | |
|---|---|---|---|
| **Item** | **Unadjusted** | **Adjusted With Forage Fiber** | **Adjusted Without Forage Fiber** |
| RD peptide ratio | 1.12 | 1.00 | 1.19 |
| RD ammonia ratio | 0.62 | 0.51 | 0.86 |
| CP % of dry matter | 14.7 | 14.4 | 16.8 |
| NEm, Mcal/lb | 1.98 | 2.07 | 2.05 |

The fermenters were fed at the rate of 30 g of dry weight/d in three equal (10g) proportions at 0800, 1600 and 2300 hr, except for the fourth treatment. For treatment four, the fermenters were fed at 80% of 30 g, or 24 g of dry weight per day, in 3 equal portions of 8 g. Diets were provided to fermenters for 7 days with measurements made the last three days. Contents from fermenters were collected and used to measure short chain fatty acids and ammonia.

Starting on days 8 and 9 of each period, two-5 ml samples were collected from each fermenter at 0, 3 and 6 hr post morning feeding. The samples were stabilized with metaphosphoric acid for short chain fatty acid analysis and with HCl for the ammonia-N analysis. The samples were stored at -20°C until analyzed for VFA (using Gas Chromatography) as described by Jenkins (1987) and ammonia-N (using Spectrophotometer) according to Cotta and Russell (1982).

On day 10 of each period, ruminal cultures from each of the four continuous fermenters were collected and strained through two layers of cheesecloth and then used within approximately 10 min after collection. Eighty milliliters of the fluid inoculum from the continuous fermenter fed the control diet was incubated with 120 ml of buffer solution (Goering and Van Soest 1970) in 250 ml ANKOM gas containing 3.0 grams of the control diet. Similarly, eighty milliliters of the fluid inoculum from each of the other 3 continuous fermenters fed the adjusted diets were incubated with 120 ml of buffer solution in 250 ml ANKOM gas plus 3.0 gram of the adjusted diet offered at a lesser feeding rate, for which each jar was provided 2.4 gram of the diet. Each treatment was run in triplicate.

Jars were gassed with CO₂ before sealing and then connected to a Tedlar gas collection bag (CEL Scientific Corp., Santa Fe Springs, CA, USA). Jars were placed into a water bath at 39 °C for 24 h. Gasses from jars were programmed to be released into connected bags when the psi exceeded 1.0. Every two hours, the jars were shaken by hand for approximately 30 seconds. After 24 h, gasbags were disconnected from jars and analyzed immediately for methane content.

From each collected gas bag, three separate gas samples were collected, using a 1 ml gas tight needle syringe (27G 1 1/4; Fisher Scientific, Chicago, IL, USA) and analyzed for methane using gas chromatography (SRI 8610C, Torrance, CA, USA) equipped with TCD detector (6" x 1/8" S.S. Shin Carbon) and ST 80/800 column (2 m x 2 mm ID). The methane peak was identified by comparing the retention time with that of the corresponding standard (Scotty Analyzed Gases 14, Sigma-Aldrich, St. Louis, MO, USA). Total gas production of the head-space sample was converted from pressure readings to mL according to Avogadro's Law equation: N = P (V/RT). The results of the study are presented in Table 18 below.

**Table 18**

| **Effects of adjusted diet on continuous culture anaerobic fermentation** | | | | |
|---|---|---|---|---|
| **Item** | **Unadjusted diet** | **Adjusted diet with forage fiber** | **Adjusted diet no forage fiber** | **Adjusted diet no forage fiber intake reduced 20%** |
| Acetic Acid (mol%) | 28.3 | 28.4 | 30.0 | 28.5 |
| Propionic Acid (mol%) | 47.6 | 47.0 | 47.2 | 46.0 |
| Isobutyric Acid (mol%) | 1.3 | 1.1 | 0.9 | 1.1 |
| Butyric Acid (mol%) | 11.5 | 13.4 | 11.4 | 14.5 |
| Isovaleric Acid (mol%) | 5.6 | 4.9 | 4.9 | 4.6 |
| Valeric Acid (mol%) | 5.7 | 5.2 | 5.6 | 5.4 |
| Total VFA (mM) | 54.3 | 60.2 | 61.5 | 45.7 |
| Ammonia-N (mM) | 5.4 | 5.0 | 3.9 | 1.4 |
| Total Gas (mL over 24 h) | 91.1 | 105.3 | 90.8 | 64.6 |
| Methane (mL over 24 h) | 8.8 | 8.7 | 6.6 | 6.5 |
| Methane, % of total gas | 9.7 | 8.3 | 7.3 | 10.0 |

The absolute amount of methane gas produced was similar for the unadjusted and adjusted diet containing forage fiber over a 24-hour period, but methane evolution was reduced by 15% when expressed as a percentage of the total gas produced (8.3% vs. 9.7%) when the adjusted diet was provided to the fermenters. Compared to the unadjusted diet, methane, and ammonia gas production both were reduced when forage fiber was removed from the adjusted diet. Reducing the intake of the adjusted diet further reduced the absolute amounts of ammonia and methane gas (mL) that were measured compared with the unadjusted diet. The reduction in ammonia evolution was attributed to protein being less degradable in the fermentation culture or to an increase in ammonia uptake and conversion to microbial protein, or both, when the adjusted diet was formulated without forage fiber.

In this experiment methane production was reduced by removing forage fiber from an adjusted diet or feeding to the fermenter a lesser amount of the adjusted diet that was devoid of forage fiber. The removal of forage fiber from adjusted diets resulted in methane production by cultures being reduced approximately 24%. Combined with improvement in efficiency of 10 to 20% attributed to lesser feed intake when adjusted diets are fed, there is the potential to reduce methane gas by 30 to 40% per unit of edible meat produced by ruminants.

### Example 10

The effect of balancing amino acid to energy ratio in diets of beef calves was assessed in this experiment. When fed diets that are not balanced to supply amino acids in relation to the requirement established by energy density of the diet, the calves will overconsume energy, have lower feed efficiencies and a lesser carcass value. Thus, the objective of this study was to determine the effect of formulating corn-based diets to meet amino acid to energy ratio on growth performance and carcass value.

**Material and Methods:** Forty-eight head of steers (BW 527 ± 3 lbs.) were utilized to examine performance and carcass characteristics. Upon arrival steers were acclimated for approximately 21 days and were fed a receiving ration. Steers were weighed on two consecutive days at the initiation and termination of the study as well as every 28 days for the entirety of the study. Weights obtained on days 1 and 0 were averaged and steers were assigned to pens based on weight such that each pen (n = 8) contained two heavy, two medium, and two light weight steers for a total of six steers per pen. Pens (n = 4/treatment) were assigned to one of two treatments: 1) grain-based ration balanced for crude protein, control **(GC);** or 2) grain-based ration balanced for amino acids (GB). All rations were balanced to meet or exceed National Research Council requirements for growing steers. Steers remained on trial till the pen achieved an average weight of 1150 - 1200 pounds. Thus, the GC and GB steers achieved the average weight goal on day 195. Upon termination of the performance trial, 5 steers per pen were shipped to a packing facility and carcass characteristics were measured and recorded.

Table 19 provides dietary compositions for the corn control diet (GC) and balanced diet (GB).

**Table 19**

| | **Corn Control Diet (GC), % as fed** | **Amino Acid-Balanced Diet (GB), % as fed** |
|---|---|---|
| Corn | 61 | 64 |
| alfalfa hay | 10 | 10 |
| beef tm | 0.05 | 0.05 |
| dried distillers grains | 26 | 16.8 |
| potassium sulfate | 0.2 | 0.2 |
| Urea | 0.6 | 0.6 |
| Lime | 1.2 | 1.4 |
| rumtm#2 | 0.1 | 0.1 |
| AjiPro^{®}-L supplement | 0.5 | 0.5 |
| Smartamine^{®} M supplement* | 0.1 | 0.1 |
| Salt | 0.25 | 0.25 |
| cottonseed meal | 0 | 6 |

| | | |
|---|---|---|
| Smartamine^{®} M supplement is a coated, rumen-protected methionine supplement (Adisseo, Alpharetta, GA) | | |

**Results:** As shown in Table 20, feed to gain ratio (FG) improved, hot carcass weight increased, and ribeye area increased in calves that received the balanced diets relative to calves that received the conventional diets.

**Table 20**

| | GC Diet | GB Diet |
|---|---|---|
| Begin weight (lb) | 526 | 530 |
| End weight (lb) | 1156 | 1168 |
| Total gain (lb) | 630 | 638 |
| Average daily gain (lb) | 3.23 | 3.29 |
| Total intake (lb) | 28181.0 | 27054.1 |
| FG (lb) | 7.47 | 7.04 |
| hot carcass weight (lb) | 711 | 726 |
| Yield Grade | 3.19 | 3.11 |
| Backfat, in | 0.52 | 0.56 |
| Ribeye area, in² | 11.71 | 12.52 |
| Dressing % | 63.7 | 63.7 |

The GB steers outperformed all other treatments with the highest ADG and the lowest feed-to-gain (FG) ratio. It took less feed for the GB steers to gain, resulting in an improved FG ratio. Steers fed GB diet had greater hot carcass weight and greater ribeye area than calves fed the control diet.

### Example 11

A trial was conducted on a commercial dairy farm to examine the benefits of feeding an adjusted diet to dairy cows. An unadjusted diet ("basal diet", "control") was prepared using a commercially available formulation program (Agricultural Modeling and Training Systems (AMTS)). The control diet was adjusted to balance for AA content relative to metabolizable energy ("optimized diet", "treated") using the disclosed technique. The trial was conducted for 42 days on a commercial dairy farm in Michigan using Holstein cows (n ≅ 450). The cows were divided into two equal groups and were balanced based upon breed, parity, stage of lactation (DIM), mean milk production and milk components measured three weeks before the co-variant adjustment period. The number of cows less than 80 DIM was equal among groups.

Cows were housed in a free stall facility and milked three times per day in a milking parlor and daily milk yields were recorded. The day 0 test data for milk yield and milk composition was used as the covariate to equalize both groups on trial.

The composition of the basal diet (control) and the treatment diet are presented in Table 21. In order to manage feed ingredient availability on the farm, the composition of diets was adjusted on day 15 of the study without affecting the testing of the optimized diet.

**Table 21**

| **Composition of diets fed to lactating cows** | | | | |
|---|---|---|---|---|
| **Ingredients (Lbs AF)** | **Control Day 0-14** | **Treatment Day 0-14** | **Control Day 15-42** | **Treatment Day 15-42** |
| Haylage | 15.3 | 15.3 | 17.8 | 17.8 |
| Corn Silage | 57.3 | 57.3 | 57.3 | 57.3 |
| Ground corn | 9.4 | 9.4 | 9.1 | 9.4 |
| Dry corn gluten feed | 6.9 | 4.1 | 6.7 | 4.1 |
| Wheat straw | 1.4 | 1.4 | 1.4 | 1.4 |
| Condensed whey | 13.7 | 13.9 | 13.3 | 13.4 |
| Canola meal | 6.9 | 6.7 | 6.7 | 6.7 |
| Specially-processed canola meal (AminoMax) | | 3.8 | | 3.8 |
| AminoPlus | 1.6 | 1.6 | 1.6 | 1.6 |
| Urea | .10 | .15 | .10 | .15 |
| AminoShure L encapsulated lysine | | .202 | | .202 |
| Minerals, vitamins, additives | 2.1 | 2.3 | 2.2 | 2.3 |
| Total | 114.7 | 116.2 | 116.2 | 118.1 |
| | | | | |
| Nutrients, DM basis | | | | |
| Dry matter, % | 50.0 | 50.0 | 49.5 | 49.5 |
| Protein, % | 15.8 | 16.9 | 15.8 | 16.8 |
| Fat, % | 3.7 | 3.8 | 3.7 | 3.8 |
| ADF, % | 18.5 | 19.1 | 19.0 | 19.6 |
| NDF, % | 29.3 | 28.7 | 29.6 | 29.1 |
| RUP, % of protein | 42.6 | 47.8 | 42.4 | 47.3 |
| RDP, % of protein | 57.4 | 52.3 | 57.8 | 52.8 |
| Soluble protein, % of protein | 42.0 | 38.0 | 42.7 | 38.8 |
| NEL, mcal/cwt | 73 | 73 | 72 | 73 |
| Lysine, grams/day | 178 | 223 | 177 | 220 |
| Methionine, grams/day | 63 | 70 | 63 | 69 |
| Histidine, grams/day | 88 | 96 | 87 | 95 |
| RUP Lys/Met | 2.43 | 3.38 | 2.45 | 3.37 |

The basal diet contained corn silage, alfalfa haylage, corn gluten, liquid whey, canola meal, vitamins, and minerals. The basal diet and the optimized diet were formulated to be iso-caloric, iso-nitrogenous and similar in RDP.

The disclosed method was used to evaluate the nutrient content and specifically the estimated supply of MP AA for the control and adjusted diets. The results of this evaluation are presented in Table 22. This evaluation indicated that the adjusted (treatment) diet was predicted to supply a more adequate amount of MP AA compared with the unadjusted (control) diet. The adjusted diet was formulated to an economically viable density of MP AA, resulting in certain MP AA being provided in lesser than 100% theoretical biologically ideal amounts.

**Table 22**

| **Evaluation of diet using disclosed model** | | |
|---|---|---|
| **Item** | **Control Diet** | **Treatment Diet** |
| Target Milk, lb/d | 90 | 90 |
| % Lactose | 4.78 | 4.78 |
| % Fat | 3.75 | 3.75 |
| % Protein | 3.10 | 3.10 |
| | | |
| NEm required, Mcal per day | 10.79 | 10.79 |
| NEl required, Mcal per day | 28.85 | 28.85 |
| NEm Intake, Mcal per day | 41.99 | 42.01 |
| NE Balance, Meal | 2.36 | 2.37 |
| Diet NE, Mcal per Kg | 1.61 | 1.61 |
| Lbs diet DM excess | 3.22 | 3.24 |
| ME Intake | 65.29 | 65.30 |
| | | |

| Amino acid balance, g per day | | |
|---|---|---|
| Met | -3.1 | 0.7 |
| Lys | -55.5 | -20.0 |
| His | 2.3 | 6.5 |
| Phe | -8.9 | 0.7 |
| Thr | -2.9 | 6.0 |
| Leu | -40.6 | -27.9 |
| Val | 7.5 | 17.9 |
| Arg | 4.2 | 17.8 |
| Ile | 7.7 | 17.1 |
| Trp | 0.1 | 2.8 |
| | | |

| Grams MP AA:Mcal ME | | |
|---|---|---|
| Met | 0.948 | 1.000 |
| Lys | 2.560 | 3.062 |
| His | 1.182 | 1.241 |
| Phe | 2.063 | 2.198 |
| Thr | 2.106 | 2.232 |
| Leu | 3.774 | 3.953 |
| Val | 2.468 | 2.615 |
| Arg | 2.410 | 2.603 |
| Ile | 2.045 | 2.179 |
| Trp | 0.544 | 0.583 |

Rations were offered as total mixed rations (TMR) once daily for ad libitum intake. Delivered diets were pushed up 8-10 times daily to encourage ad libitum dry matter intake (DMI). The amount of feed offered was adjusted to allow for about five percent feed refusals. Feed refusals were measured and recorded daily. The number of animals in each group and pen daily milk production were recorded daily to enable calculation of a pen mean for DMI per cow per day. The TMR were mixed and delivered with a vertical feed mixer (Kuhn-Knight, vertical Maxx VT 180). Feed DM was monitored daily and adjustments in amounts of dry weight of feed offered were made accordingly. Feed offered and estimated consumption (offered-refusal) were recorded daily.

Feed samples were collected weekly and dry matters determined and used to make adjustment in the rations (as-fed basis). Samples of individual feeds collected at the beginning were analyzed for nutrient analysis. Samples of diets were collected weekly and a composite sample formed for nutrient analysis to verify the nutrient profile of the diets consumed. Samples of final ration were collected weekly and a composite sample was analyzed for fiber, protein, and energy. Individual milk weights were recorded by pen at each milking for all animals. Milk protein, fat, lactose, somatic cell counts, and milk urea nitrogen were determined by standard methods.

Data was analyzed using a repeated measures, completely randomized design. Yields of milk and milk components were co-variant adjusted using data collected in the Day 0 test period. The statistical model included DIM to account for the effects of lactation stage. Significance was declared at P<0.10 unless otherwise stated.

The results of the study are presented in Table 23. The treatment diet was demonstrated as superior to the control diet relative to total production of milk per day, milk protein %, milk protein yield, lactose %, non-fat solids % and somatic cell counts for the entire test. The increased milk production did cause the treatment cows to increase their dry matter intake (DMI) numerically over the control cows; however, this increase difference was minimal (57.2 lb of DM/cow/day for the control cows versus 57.8 lb of DM/cow/day for the treatment cows). In general, the treatment cows performed better than control cows. The results of this study demonstrated the benefits for using the disclosed method to optimize diets fed to dairy cows on a commercial dairy farm.

**Table 23**

| **Milk production and composition for lactating cows** | | | | | | | |
|---|---|---|---|---|---|---|---|
| ITEM | | | TREATMENTS | | SEM | *P VALUE* | |
| | | | 1 | 2 | | Trt | Treat x week |
| Milk Yeld | | | | | | | |
| | Milk, lbs/d | | 97.3 | 101.2 | 1.68 | 0.09 | 0.58 |

| Milk components | | | | | | | |
|---|---|---|---|---|---|---|---|
| | Protein, % | | 2.88 | 2.96 | 0.016 | <0.01 | <0.01 |
| | | Protein lbs/d | 2.88 | 3.09 | 0.063 | 0.02 | 0.43 |
| | Fat, % | | 3.42 | 3.40 | 0.042 | 0.67 | 0.29 |
| | | Fat lbs/d | 3.42 | 3.53 | 0.087 | 0.37 | 0.28 |
| | Lactose % | | 4.85 | 4.90 | 0.008 | <0.01 | 0.79 |
| | | Lactose lbs/d | 4.91 | 5.17 | 0.122 | 0.12 | 0.32 |
| | Solids not-fat, % | | 5.81 | 5.86 | 0.009 | <0.01 | 0.05 |
| | | Solids not-fat, lbs/d | 5.87 | 6.19 | 0.144 | 0.11 | 0.35 |
| | SCC x 1,000 cells/mL | | 127 | 60 | 21.8 | 0.02 | 0.98 |
| | ECM, Kg/d | | 41.0 | 43.0 | 0.97 | 0.15 | 0.32 |

### Example 12

A study was performed to assess the impact of eliminating roughage from the diet of cattle. One group of cattle were fed diets that included 6% cottonseed hulls (CSH), while the other group of cattle were fed diets that had no roughage added. In the art, the current understanding is that cattle cannot be fed diets without added roughage. However, as reflected in the preliminary data show in Table 24, the removal of roughage from the diet while otherwise optimizing the diet to meet the cattle's amino acid requirements, as described herein, results in a comparable average daily gain (ADG) at a lower overall feed cost. Note that the greater ADG for the roughage-added group as compared to the no-roughage-added group may have been due to one pen having an elevated ADG of 4.7 lbs, and this value may decrease as the cattle in that pen get heavier.

**Table 24**

| **Preliminary data comparing cattle attributes on a no-roughage added diet** | | | | | | | | |
|---|---|---|---|---|---|---|---|---|
| | **BODY WEIGHT (LBS)** | | | | | | | |
| **Diet** | **Starting** | **Midpoint** | **As Fed Intake** | **DM Intake (88%)** | **ADG** | **Feed-to-gain (FG)** | **Feed cost/day** | **Gain value @ $0.99** |
| No added roughage (no CSH) | 522 | 923 | 22.0 | 19.4 | 4.0 | 4.8 | $ 1.88 | $ 3.96 |
| Added roughage (6% CSH) | 511 | 950 | 25.2 | 22.2 | 4.4 | 5.0 | $ 2.32 | $ 4.36 |
| Difference | | | 12.7% | | 9% | 4% | +$ 0.44 | -$ 0.40 |

In particular, roughage removal reduced intake 12.7%, reduced daily feed costs by $0.44, reduced daily gain 9%, improved feed to gain 4%, and reduced daily value of gain $0.40 per day assuming live weight value of $0.99 per pound. The net difference from roughage removal increased the value of each calf by $0.04 per day, thereby yielding an advantage of $8.80 per calf over 220 day feeding period. It is also possible to maintain the no-roughage-added cattle in the lot for longer, e.g., 10 additional days, such that the no-roughage-added cattle achieves the same final weight as the roughage-added cattle. While the extension to the feeding period would add about $20 in feed costs (and associated feed lot costs), the extension would yield an additional $40 in revenue based on the additional weight (at least based on the $0.99 per pound price). The no-roughage-added group is also expected to yield an improved carcass value as compared to the roughage-added group.

### Example 13

A continuous culture study was conducted to evaluate short chain fatty acid evolution and methane gas production when the fiber content was reduced in adjusted diets and when the culture system was fed lesser amounts of the adjusted diet.

Optimizing a dietary amino acid profile to balance for diet energy density (AA:ME) necessitates that dietary protein and/or amino acids be altered. Altering dietary the protein source and level can affect protein and nitrogen available to rumen microflora. Because diets are formulated to meet rumen microflora requirements for protein and nitrogen no effect on rumen fermentation characteristics should occur, with methane production being similar between a high starch (corn) diet unbalanced or balanced to meet the animal's amino acid requirement. However, the ability to formulate diets to meet the AA:ME requirement allows high starch diets to be fed to beef cattle without inclusion of forage fiber in the diet to maintain rumen health. Removing forage from a high starch diet would be expected to create a different structure of rumen digesta, specifically formation of a mat layer would not be expected to occur. A mat forms from the less dense forage material floating on top of the rumen digesta which is a fluid (water) suspension. The mat creates an environment where protozoa and methanogenic bacteria reside. Lack of mat formation would reduce a sustaining environment for methanogenic bacteria and potentially reduce methane production.

A continuous culture experiment was conducted to determine the effect of balancing a typical commercial diet for AA:ME with forage fiber on methane production and to determine the effect of forage removal from an AA:ME balanced diet on methane production.

Rumen fluid containing an active rumen fermentation culture was collected from a steer fed a typical commercial feedlot diet not formulated to balance AA:ME. The culture was used to inoculate continuous culture fermenters fed the typical commercial feedlot diet containing 8% forage fiber (control), the control diet with protein adjusted to balance AA:ME ratios (treatment), a diet balanced for AA:ME with the forage fiber removed (no roughage) and the no roughage diet fed to fermenters at 80% of the mass fed for the no roughage treatment.

Diets were fed to fermenters for 7 days with measurements made the last three days. Contents from fermenters were collected and used to measure short chain fatty acids and ammonia. As hypothesized, there were no differences between short chain acid production or ammonia level between control and treatment diets. Forage fiber removal from diets didn't affect short chain fatty acid production but did result in less nitrogen loss as evidenced by reduced ammonia concentration in fermenter contents (28% reduction). When intake was reduced to 80% short chain fatty acid production from fermentation was reduced as expected due to reduced mass of fermentable substrate.

These data show that fermentation characteristics are altered by formulating diets for AA:ME and removing forage fiber from diets. Balancing diets for AA:ME reduces fermentation output linearly by the amount of improved efficiency (reduced intake per unit of output) gained from meeting amino acid requirements for growth and production. Forage fiber removal from diets further reduces ammonia loss compared to control diets.

Of primary interest in this example is the impact on methane production when diets are formulated to meet AA:ME requirement. Typical improvements in efficiency that occur from formulating diets to meet amino acid requirement range from 5 to 20%. Formulating typical beef feedlot diets to meet amino acid requirement reduces methane production linearly by the same level of efficiency improvement. Removing forage fiber from the diet, which is possible if diets are balanced to meet the animal's amino acid requirement, reduced methane production 26% compared to fermentation of the control diet. Formulating ruminant diets for AA:ME reduced methane production by rumen microbes 26%.

**Table 25**

| **Effect of Balancing Diets to Meet Amino Acid Requirement and Roughage Removal on Ammonia and Methane Production from a Rumen Fermentation of Starch-Based Diets** | | | | |
|---|---|---|---|---|
| **Item** | **Control Diet** | **AA Balanced with Roughage** | **AA Balanced with no roughage** | **AA Balanced with no roughage fed at 80% Intake** |
| Acetic Acid (mol%) | 28.3 | 28.4 | 30.0 | 28.5 |
| Propionic Acid (mol%) | 47.6 | 47.0 | 47.2 | 46.0 |
| Isobutyric Acid (mol%) | 1.3 | 1.1 | 0.9 | 1.1 |
| Butyric Acid (mol%) | 11.5 | 13.4 | 11.4 | 14.5 |
| Isovaleric Acid (mol%) | 5.6 | 4.9 | 4.9 | 4.6 |
| Valeric Acid (mol%) | 5.7 | 5.2 | 5.6 | 5.4 |
| Total VFA (mM) | 54.3 | 60.2 | 61.5 | 45.7 |
| Ammonia-N (mM) | 5.4 | 5.0 | 3.9 | 1.4 |
| Total Gas (mL over 24 h) | 91.1 | 105.3 | 90.8 | 64.6 |
| Methane (mL over 24 h) | 8.8 | 8.7 | 6.6 | 6.5 |

## Claims

1. A method, comprising:
determining an amount of urea, a protein source, peptides, rumen-protected peptides, rumen-protected amino acids, or any combination thereof, to add to an initial ruminant diet to provide an amino acid-balanced diet that satisfies a ruminant's amino acid requirement, wherein
the amount is based at least in part on an amino acid comparison between (i) a prediction of dietary amino acid and microbial amino acid flow to the ruminant's small intestine, and (ii) the ruminant's amino acid requirement; and
the ruminant's amino acid requirement is based at least in part upon usable energy consumed by the ruminant and an energy requirement of the ruminant, wherein (i) the usable energy is effective energy and the energy requirement is an effective energy requirement based on the ruminant's requirements for maintenance heat energy, protein accretion energy, lipid accretion energy, and methane gas energy, or (ii) the usable energy is metabolizable energy and the energy requirement is a metabolizable energy requirement based on the ruminant's requirements for maintenance heat energy, protein accretion energy, lipid accretion energy, methane gas energy, and lactation energy; and
at least part of the method is performed by one or more computing devices.

2. The method of claim 1, further comprising:
preparing an adjusted diet, a dietary supplement, or a combination thereof, comprising the amount of urea, protein source, peptides, rumen-protected peptides, rumen-protected amino acids, or combination thereof,
preferably wherein
(i) the dietary supplement comprises rumen-protected amino acids, optionally wherein the rumen-protected amino acids are essential amino acids; or
(ii) the dietary supplement comprises a protein source, peptides, rumen-protected peptides, or a combination thereof; or
(iii) the adjusted diet comprises an additional protein source, a different protein source, an adjusted amount of a protein source, a reduced amount of roughage, or a combination thereof, relative to the initial diet.

3. The method of claim 1 or claim 2, further comprising:
(i) determining a quantity of usable energy provided by an amount of the initial diet consumed by the ruminant, preferably wherein determining the quantity of usable energy further comprises determining protein, starch, and fiber content of the initial diet; or
(ii) determining the usable energy requirement of the ruminant; or
(iii) determining the amino acid requirement of the ruminant based at least in part on the energy requirement for the ruminant and the quantity of usable energy provided by the amount of the initial diet consumed by the ruminant; or
(iv) determining ruminal microbial efficiency, preferably wherein determining ruminal microbial efficiency is based at least in part on dilution rate; or
(v) determining (a) a rumen microbial peptide-nitrogen or amino acid-nitrogen requirement and (b) a rumen microbial ammonia-nitrogen requirement; or
(vi) any combination of (i), (ii), (iii), (iv), and (v).

4. The method of claim 3, further comprising:
comparing peptide-nitrogen or amino acid-nitrogen supplied by the initial diet to the microbial peptide-nitrogen or amino acid-nitrogen requirement to provide a peptide-nitrogen or amino acid-nitrogen comparison; and
comparing ammonia-nitrogen supplied by the initial diet to the microbial ammonia-nitrogen requirement to provide an ammonia-nitrogen comparison,
preferably wherein determining the amount of urea, protein source, peptides, rumen-protected amino acids, or any combination thereof, is further based at least in part on the peptide-nitrogen or amino acid-nitrogen comparison and the ammonia-nitrogen comparison.

5. The method of any one of claims 2-4, wherein:
the usable energy is effective energy, and the adjusted diet, the dietary supplement, or the combination thereof provides grams of essential amino acids per megajoule of effective energy within ranges of Lys 0.2-2, Met 0.05-1, His 0.1-1, Phe > 0.2, Thr > 0.2, Leu > 0.4, Val > 0.2, Arg 0.2-2, Ile > 0.2, Trp > 0.03, or any combination thereof for growing animals, preferably within ranges of Lys 0.4-0.8, Met 0.12-0.25, His 0.15-0.35, Phe ≥ 0.2, Thr ≥ 0.25, Leu ≥ 0.45, Val ≥ 0.25, Arg 0.4-0.8, Ile ≥ 0.2, Trp ≥ 0.03, or any combination thereof; or
the usable energy is metabolizable energy, and the adjusted diet, the dietary supplement, or the combination thereof provides grams of essential amino acids per megajoule of metabolizable energy within ranges of Lys 0.2-2.5, Met 0.1-0.6, His 0.1-1, Phe ≥ 1.5, Thr ≥ 0.2, Leu ≥ 0.7, Arg ≥ 0.3, Ile ≥ 0.3, Trp ≥ 0.07, or any combination thereof for lactating animals, preferably within ranges of Lys 0.7-1, Met 0.2-0.3, His 0.25-0.35, Phe ≥ 0.4, Thr ≥ 0.35, Leu ≥ 0.84, Val ≥ 0.47, Arg ≥ 0.43, Ile ≥ 0.38, Trp ≥ 0.09, or any combination thereof.

6. A system, comprising:
one or more processors; and
a computer-readable storage media storing computer-executable instructions that, when executed by the one or more processors, cause the one or more processors to collectively carry out the method of claim 1.

7. The system of claim 6, wherein the computer-readable storage media store additional computer-executable instructions that, when executed by the one or more processors, cause the one or more processors to collectively carry out the method of claim 3.

8. The system of claim 7, wherein the computer-readable storage media store additional computer-executable instructions that, when executed by the one or more processors:
(i) cause the one or more processors to collectively determine the prediction of dietary amino acid and microbial amino acid flow to the ruminant's small intestine based at least in part on the ruminal microbial efficiency, a quantity of usable energy provided by an amount of the initial diet consumed by the ruminant, and the protein, starch, and fiber content of the initial diet;
or
(ii) cause the one or more processors to carry out the method of claim 4; or
(iii) both (i) and (ii).

9. The system of any of claims 6-8, wherein the computer-readable storage media collectively store additional computer-executable instructions that, when executed by the one or more processors:
(i) cause the one or more processors to collectively perform further operations comprising:
(c1) receiving one or more first signals from an initial diet formulation system, the one or more first signals conveying data regarding an initial diet formulation for the ruminant; or
(c2) receiving one or more second signals conveying data regarding the ruminant; or
(c3) receiving one or more third signals conveying data regarding an available feed product, an available dietary supplement, or combination thereof; or
(c4) any combination of (c1), (c2), and (c3); or
(ii) cause the one or more processors to collectively select one or more feed products, one or more dietary supplements, or any combination thereof that, in combination with the initial ruminant diet, satisfy the determined amount of urea, protein source, peptides, rumen-protected peptides, rumen-protected amino acids, or any combination thereof; or
(iii) both (i) and (ii).

10. The system of claim 9, wherein:
the computer-readable storage media store additional computer-executable instructions that, when executed by the one or more processors, cause the one or more processors to collectively generate one or more fourth signals based at least in part on the selected one or more feed products, one or more dietary supplements, or combination thereof, and
the one or more fourth signals causes:
(d1) a user interface to display the selected one or more feed products, one or more dietary supplements, or combinations thereof; or
(d2) a feed dispenser to automatically adjust a diet fed to the ruminant to include the selected one or more feed products, one or more dietary supplements, or combination thereof; or
(d3) both (d1) and (d2).

11. The system of any of claims 6-10, further comprising an input/output interface situated to:
(e1) electronically communicate with an initial diet formulation system that determines the initial ruminant diet; or
(e2) electronically communicate with a memory or database storing data regarding available feed and dietary supplement products for the ruminant; or
(e3) electronically communicate with a user interface comprising an input/output device, a display, or both; or
(e4) electronically communicate with and/or automatically control a feed system that delivers feed to the ruminant; or
(e5) any combination of (e1), (e2), (e3), and (e4).

12. The system of any of claims 6-11, wherein the computer-readable storage media store additional computer-executable instructions that, when executed by the one or more processors, cause the one or more processors to collectively generate one or more control signals for a feed system that cause the feed system to prepare an adjusted diet, a dietary supplement, or combination thereof, comprising the determined amount of urea, protein source, peptides, rumen-protected peptides, rumen-protected amino acids, or combination thereof.

13. One or more non-transitory computer-readable media collectively storing computer-executable instructions that, when executed by one or more computing devices, configure the one or more computing devices to perform at least part of the method of claim 1.

14. The one or more non-transitory computer-readable media of claim 13, wherein the instructions stored by the one or more non-transitory computer-readable media comprise:
(a1) instructions that cause the one or more computing devices to receive one or more first signals conveying data regarding an initial diet formulation for the ruminant; or
(a2) instructions that cause the one or more computing devices to receive one or more second signals conveying data regarding the ruminant; or
(a3) instructions that cause the one or more computing devices to receive one or more third signals conveying data regarding an available feed product, an available dietary supplement, or combination thereof; or
(a4) any combination of (a1), (a2), and (a3).

15. The one or more non-transitory computer-readable media of claim 13 or claim 14, wherein the instructions stored by the one or more non-transitory computer-readable media comprise instructions that cause the one or more computing devices to select one or more feed products, one or more dietary supplements, or any combination thereof based, at least in part, on the determined amount of urea, protein source, peptides, rumen-protected peptides, rumen-protected amino acids, or any combination thereof.

16. The one or more non-transitory computer-readable media of any one of claims 13-15, wherein:
the instructions stored by the one or more non-transitory computer-readable media comprise instructions that cause the one or more computing devices to transmit one or more fourth signals, and
the one or more fourth signals causes
(b1) a user interface to display the determined amount of urea, protein source, peptides, rumen-protected peptides, rumen-protected amino acids, or any combination thereof; or
(b2) a user interface to display the selected one or more feed products, one or more dietary supplements, or any combination thereof; or
(b3) a feed dispenser to adjust a diet fed to the ruminant to include the selected one or more feed products, one or more dietary supplements, or combination thereof; or
(b4) any combination of (b1), (b2), (b3), and (b4).

## Patentansprüche

1. Verfahren, das umfasst:
Bestimmen einer Menge an Harnstoff, einer Proteinquelle, Peptiden, pansenstabilen Peptiden, pansenstabilen Aminosäuren oder einer beliebigen Kombination davon, die einer Ausgangsdiät für Wiederkäuer beigemischt wird, um eine aminosäureausgewogene Diät bereitzustellen, die den Aminosäurebedarf eines Wiederkäuers deckt, wobei
die Menge zumindest teilweise auf einem Aminosäurevergleich zwischen (i) einer Vorhersage des Flusses von Nahrungsaminosäuren und mikrobiellen Aminosäuren in den Dünndarm des Wiederkäuers und (ii) dem Aminosäurebedarf des Wiederkäuers basiert; und
der Aminosäurebedarf des Wiederkäuers zumindest teilweise auf der vom Wiederkäuer aufgenommenen verwertbaren Energie und einem Energiebedarf des Wiederkäuers basiert, wobei (i) die verwertbare Energie die effektive Energie ist und der Energiebedarf ein effektiver Energiebedarf ist, der auf den Anforderungen des Wiederkäuers an Wärmeenergie zur Erhaltung, Energie für den Proteinabbau, Energie für den Lipidabbau und Energie für die Methanbildung basiert, oder (ii) die verwertbare Energie metabolisierbare Energie ist und der Energiebedarf ein metabolisierbarer Energiebedarf ist, der auf den Anforderungen des Wiederkäuers an Wärmeenergie zur Erhaltung, Energie für den Proteinabbau, Energie für den Lipidabbau, Methangasenergie und Laktationsenergie basiert; und
zumindest ein Teil des Verfahrens von einer oder mehreren Computervorrichtungen durchgeführt wird.

2. Verfahren nach Anspruch 1, das ferner umfasst:
Zubereiten einer angepassten Diät, eines Nahrungsergänzungsmittels oder einer Kombination davon, die die Menge an Harnstoff, Proteinquelle, Peptiden, pansenstabilen Peptiden, pansenstabilen Aminosäuren oder einer Kombination davon umfasst,
wobei vorzugsweise
(i) das Nahrungsergänzungsmittel pansenstabile Aminosäuren umfasst, optional wobei die pansenstabilen Aminosäuren essentielle Aminosäuren sind; oder
(ii) das Nahrungsergänzungsmittel eine Proteinquelle, Peptide, pansenstabile Peptide oder eine Kombination davon enthält; oder
(iii) die angepasste Diät eine zusätzliche Proteinquelle, eine andere Proteinquelle, eine angepasste Menge einer Proteinquelle, eine reduzierte Menge an Raufutter oder eine Kombination davon im Vergleich zur Ausgangsdiät enthält.

3. Verfahren nach Anspruch 1 oder Anspruch 2, das ferner umfasst:
(i) Bestimmen einer Menge an nutzbarer Energie, die durch eine vom Wiederkäuer aufgenommene Menge der Ausgangsdiät bereitgestellt wird, wobei das Bestimmen der Menge an nutzbarer Energie vorzugsweise ferner das Bestimmen des Protein-, Stärke- und Ballaststoffgehalts der Ausgangsdiät umfasst; oder
(ii) Bestimmen des Bedarfs an nutzbarer Energie des Wiederkäuers; oder
(iii) Bestimmen des Aminosäurebedarfs des Wiederkäuers, zumindest teilweise basierend auf dem Energiebedarf des Wiederkäuers und der Menge an nutzbarer Energie, die durch die vom Wiederkäuer aufgenommene Menge der Ausgangsdiät bereitgestellt wird; oder
(iv) Bestimmen der mikrobiellen Effizienz im Pansen, vorzugsweise wobei das Bestimmen der mikrobiellen Effizienz im Pansen zumindest teilweise auf der Verdünnungsrate basiert; oder
(v) Bestimmen (a) eines Bedarfs an Peptidstickstoff oder Aminosäure-Stickstoff der Pansenmikroorganismen und (b) eines Ammoniak-Stickstoff-Bedarfs der Pansenmikroorganismen; oder
(vi) eine beliebige Kombination aus (i), (ii), (iii), (iv) und (v).

4. Verfahren nach Anspruch 3, das ferner umfasst:
Vergleichen des durch die Ausgangsdiät zugeführten Peptid-Stickstoffs oder Aminosäure-Stickstoffs mit dem mikrobiellen Peptid-Stickstoff- oder Aminosäure-Stickstoff-Bedarf, um einen Peptid-Stickstoff- oder Aminosäure-Stickstoff-Vergleich zu erhalten; und
Vergleichen des durch die Ausgangsdiät zugeführten Ammoniak-Stickstoffs mit dem mikrobiellen Ammoniak-Stickstoff-Bedarf, um einen Ammoniak-Stickstoff-Vergleich zu erhalten,
wobei vorzugsweise das Bestimmen der Menge an Harnstoff, Proteinquelle, Peptiden, pansenstabilen Aminosäuren oder einer beliebigen Kombination davon zumindest teilweise auf dem Peptidstickstoff- oder Aminosäure-Stickstoff-Vergleich und dem Ammoniak-Stickstoff-Vergleich basiert.

5. Verfahren nach einem der Ansprüche 2-4, wobei:
die nutzbare Energie die effektive Energie ist und die angepasste Diät, das Nahrungsergänzungsmittel oder deren Kombination Gramm essenzieller Aminosäuren pro Megajoule effektiver Energie in folgenden Bereichen bereitstellt: Lys 0,2-2, Met 0,05-1, His 0,1-1, Phe > 0,2, Thr > 0,2, Leu > 0,4, Val > 0,2, Arg 0,2-2, Ile > 0,2, Trp > 0,03 oder eine beliebige Kombination davon für wachsende Tiere, vorzugsweise in den Bereichen von Lys 0,4-0,8, Met 0,12-0,25, His 0,15-0,35, Phe ≥ 0,2, Thr ≥ 0,25, Leu ≥ 0,45, Val ≥ 0,25, Arg 0,4-0,8, Ile ≥ 0,2, Trp ≥ 0,03 oder eine beliebige Kombination davon; oder
die nutzbare Energie die verwertbare Energie ist und die angepasste Diät, das Nahrungsergänzungsmittel oder deren Kombination Gramm essenzieller Aminosäuren pro Megajoule verwertbarer Energie in folgenden Bereichen bereitstellt: Lys 0,2-2,5, Met 0,1-0,6, His 0,1-1, Phe ≥ 1,5, Thr ≥ 0,2, Leu ≥ 0,7, Arg ≥ 0,3, Ile ≥ 0,3, Trp ≥ 0,07 oder einer beliebigen Kombination davon für laktierende Tiere, vorzugsweise in den Bereichen Lys 0,7-1, Met 0,2-0,3, His 0,25-0,35, Phe ≥ 0,4, Thr ≥ 0,35, Leu ≥ 0,84, Val ≥ 0,47, Arg ≥ 0,43, Ile ≥ 0,38, Trp ≥ 0,09 oder eine beliebige Kombination davon.

6. System, das umfasst:
einen oder mehrere Prozessoren; und
ein computerlesbares Speichermedium, auf dem computerausführbare Befehle gespeichert sind, die, wenn sie von dem einen oder den mehreren Prozessoren ausgeführt werden, bewirken, dass der eine oder die mehreren Prozessoren gemeinsam das Verfahren nach Anspruch 1 ausführen.

7. System nach Anspruch 6, wobei das computerlesbare Speichermedium zusätzliche computerausführbare Befehle speichert, die, wenn sie von dem einen oder den mehreren Prozessoren ausgeführt werden, bewirken, dass der eine oder die mehreren Prozessoren gemeinsam das Verfahren nach Anspruch 3 ausführen.

8. System nach Anspruch 7, wobei das computerlesbare Speichermedium zusätzliche computerausführbare Befehle speichert, die, wenn sie von dem einen oder den mehreren Prozessoren ausgeführt werden:
(i) bewirken, dass der eine oder die mehreren Prozessoren gemeinsam die Vorhersage des Flusses von Aminosäuren aus der Nahrung und von mikrobiellen Aminosäuren in den Dünndarm des Wiederkäuers ermitteln, zumindest teilweise basierend auf der mikrobiellen Effizienz im Pansen, einer Menge an nutzbarer Energie, die durch eine vom Wiederkäuer aufgenommene Menge der Ausgangsdiät bereitgestellt wird, sowie dem Protein-, Stärke- und Ballaststoffgehalt der Ausgangsdiät; oder
(ii) bewirken, dass der eine oder die mehreren Prozessoren das Verfahren nach Anspruch 4 ausführen; oder
(iii) sowohl (i) als auch (ii).

9. System nach einem der Ansprüche 6-8, wobei das computerlesbare Speichermedium gemeinsam zusätzliche computerausführbare Befehle speichert, die, wenn sie von dem einen oder den mehreren Prozessoren ausgeführt werden:
(i) bewirken, dass der eine oder die mehreren Prozessoren gemeinsam weitere Operationen ausführen, die umfassen:
(c1) Empfangen eines oder mehrerer erster Signale von einem System zur Formulierung der Ausgangsdiät, wobei das eine oder die mehreren ersten Signale Daten bezüglich einer Ausgangsdiät für Wiederkäuer übermitteln; oder
(c2) Empfangen eines oder mehrerer zweiter Signale, die Daten bezüglich des Wiederkäuers übermitteln; oder
(c3) Empfangen eines oder mehrerer dritter Signale, die Daten bezüglich eines verfügbaren Futtermittels, eines verfügbaren Nahrungsergänzungsmittels oder einer Kombination davon übermitteln; oder
(c4) eine beliebige Kombination aus (c1), (c2) und (c3); oder
(ii) bewirkten, dass der eine oder die mehreren Prozessoren gemeinsam ein oder mehrere Futtermittel, ein oder mehrere Nahrungsergänzungsmittel oder eine beliebige Kombination davon auswählen, die in Kombination mit der Ausgangsdiät für Wiederkäuer die ermittelte Menge an Harnstoff, Proteinquelle, Peptiden, pansenstabilen Peptiden, pansenstabilen Aminosäuren oder einer beliebigen Kombination davon erfüllen; oder
(iii) sowohl (i) als auch (ii).

10. System nach Anspruch 9, wobei:
das computerlesbare Speichermedium zusätzliche computerausführbare Befehle speichert, die, wenn sie von dem einen oder den mehreren Prozessoren ausgeführt werden, bewirken, dass der eine oder die mehreren Prozessoren gemeinsam ein oder mehrere vierte Signale erzeugen, die zumindest teilweise auf dem ausgewählten einen oder den mehreren Futtermitteln, einem oder mehreren Nahrungsergänzungsmitteln oder einer Kombination davon basieren, und
das eine oder die mehreren vierten Signale bewirken:
(d1) dass eine Benutzerschnittstelle das ausgewählte eine oder mehrere Futtermittel, das ausgewählte eine oder mehrere Nahrungsergänzungsmittel oder Kombinationen davon anzeigt; oder
(d2) dass ein Futterausgeber die dem Wiederkäuer verabreichte Diät automatisch so anpasst, dass sie das ausgewählte eine oder mehrere Futtermittel, das ausgewählte eine oder mehrere Nahrungsergänzungsmittel oder Kombinationen davon enthält; oder
(d3) sowohl (d1) als auch (d2).

11. System nach einem der Ansprüche 6-10, das ferner eine Eingabe-/Ausgabe-Schnittstelle umfasst, die für Folgendes ausgelegt ist:
(e1) elektronisches Kommunizieren mit einem System zur Erstellung einer Ausgangsdiätsformulierung, das die Ausgangsdiät für Wiederkäuer bestimmt; oder
(e2) elektronisches Kommunizieren mit einem Speicher oder einer Datenbank, in dem bzw. der Daten zu verfügbaren Futter- und Nahrungsergänzungsmitteln für Wiederkäuer gespeichert sind; oder
(e3) elektronisches Kommunizieren mit einer Benutzerschnittstelle, die ein Ein-/Ausgabegerät, eine Anzeige oder beides umfasst; oder
(e4) elektronisches Kommunizieren mit einem Fütterungssystem und/oder automatisches Steuern desselben, das dem Wiederkäuer Futter zuführt; oder
(e5) eine beliebige Kombination aus (e1), (e2), (e3) und (e4).

12. System nach einem der Ansprüche 6-11, wobei das computerlesbare Speichermedium zusätzliche computerausführbare Befehle speichert, die, wenn sie von dem einen oder den mehreren Prozessoren ausgeführt werden, bewirken, dass der eine oder die mehreren Prozessoren gemeinsam ein oder mehrere Steuersignale für ein Fütterungssystem erzeugt, die das Fütterungssystem dazu veranlassen, eine angepasste Diät, ein Nahrungsergänzungsmittel oder eine Kombination davon zuzubereiten, die die ermittelte Menge an Harnstoff, Proteinquelle, Peptiden, pansenstabilen Peptiden, pansenstabilen Aminosäuren oder einer Kombination davon enthält.

13. Ein oder mehrere nicht-flüchtige, computerlesbare Speichermedien, die gemeinsam computerausführbare Befehle speichern, die, wenn sie von einer oder mehreren Computervorrichtungen ausgeführt werden, die eine oder mehreren Computervorrichtungen so konfigurieren, dass sie zumindest einen Teil des Verfahrens nach Anspruch 1 ausführen.

14. Ein oder mehrere nicht-flüchtige, computerlesbare Speichermedien nach Anspruch 13, wobei die auf dem einen oder den mehreren nicht-flüchtigen, computerlesbaren Speichermedien gespeicherten Befehle Folgendes umfassen:
(a1) Befehle, die bewirken, dass die eine oder mehreren Computervorrichtungen ein oder mehrere erste Signale empfangen, die Daten bezüglich einer Ausgangsdiätzusammensetzung für Wiederkäuer übermitteln; oder
(a2) Befehle, die bewirken, dass die eine oder mehreren Computervorrichtungen ein oder mehrere zweite Signale empfangen, die Daten bezüglich des Wiederkäuers übermitteln; oder
(a3) Befehle, die bewirken, dass die eine oder mehreren Computervorrichtungen ein oder mehrere dritte Signale empfangen, die Daten bezüglich eines verfügbaren Futtermittels, eines verfügbaren Nahrungsergänzungsmittels oder einer Kombination davon übermitteln; oder
(a4) eine beliebige Kombination aus (a1), (a2) und (a3).

15. Ein oder mehrere nicht-flüchtige, computerlesbare Speichermedien nach Anspruch 13 oder Anspruch 14, wobei die auf dem einen oder den mehreren nicht-flüchtigen, computerlesbaren Speichermedien gespeicherten Befehle Befehle umfassen, die die eine oder die mehreren Computervorrichtungen veranlassen, ein oder mehrere Futtermittel, ein oder mehrere Nahrungsergänzungsmittel oder eine beliebige Kombination davon auszuwählen, zumindest teilweise basierend auf der ermittelten Menge an Harnstoff, Proteinquelle, Peptiden, pansenstabilen Peptiden, pansenstabilen Aminosäuren oder einer beliebigen Kombination davon.

16. Ein oder mehrere nicht-flüchtige, computerlesbare Medien nach einem der Ansprüche 13-15, wobei:
die auf dem einen oder den mehreren nicht-flüchtigen, computerlesbaren Speichermedien gespeicherten Befehle Befehle umfassen, die bewirken, dass die eine oder mehreren Computervorrichtungen ein oder mehrere vierte Signale übertragen, und
das eine oder die mehreren vierten Signale bewirken,
(b1) dass eine Benutzerschnittstelle die ermittelte Menge an Harnstoff, Proteinquelle, Peptiden, pansenstabilen Peptiden, pansenstabilen Aminosäuren oder einer beliebigen Kombination davon anzeigt; oder
(b2) dass eine Benutzerschnittstelle das ausgewählte eine oder mehrere Futtermittel, das ausgewählte eine oder mehrere Nahrungsergänzungsmittel oder eine beliebige Kombination davon anzeigt; oder
(b3) dass ein Futterausgeber die dem Wiederkäuer verabreichte Diät so anpasst, dass sie das ausgewählte eine oder mehrere Futtermittel, das ausgewählte eine oder mehrere Nahrungsergänzungsmittel oder Kombinationen davon enthält; oder
(b4) eine beliebige Kombination aus (b1), (b2), (b3) und (b4).

## Revendications

1. Procédé comprenant :
la détermination d'une quantité d'urée, d'une source de protéines, de peptides, de peptides protégés contre la dégradation ruminale, d'acides aminés protégés contre la dégradation ruminale ou de toute combinaison de ceux-ci, à ajouter à un régime alimentaire initial pour ruminants afin de fournir un régime alimentaire équilibré en acides aminés qui satisfasse le besoin en acides aminés d'un ruminant ;
la quantité reposant au moins en partie sur une comparaison des acides aminés entre (i) une prédiction du flux d'acides aminés alimentaires et d'acides aminés microbiens vers l'intestin grêle du ruminant, et (ii) le besoin en acides aminés du ruminant, et
le besoin en acides aminés du ruminant reposant au moins en partie sur l'énergie utilisable consommée par le ruminant et sur le besoin en énergie du ruminant, étant entendu que (i) l'énergie utilisable est l'énergie effective et le besoin en énergie est un besoin en énergie effectif fonction des besoins du ruminant en énergie calorique d'entretien, en énergie d'accrétion protéique, en énergie d'accrétion lipidique et en énergie de méthane, ou (ii) l'énergie utilisable étant une énergie métabolisable et le besoin en énergie étant un besoin en énergie métabolisable fonction des besoins du ruminant en énergie calorique d'entretien, en énergie d'accrétion protéique, en énergie d'accrétion lipidique, en énergie de méthane et en énergie de lactation, et
au moins une partie du procédé étant réalisée par un ou plusieurs dispositifs informatiques.

2. Procédé selon la revendication 1, comprenant en outre :
la préparation d'un régime alimentaire ajusté, d'un complément alimentaire ou d'une combinaison de ceux-ci, comprenant la quantité d'urée, de source de protéines, de peptides, de peptides protégés contre la dégradation ruminale, d'acides aminés protégés contre la dégradation ruminale ou d'une combinaison de ceux-ci ;
et dans lequel de préférence :
(i) le complément alimentaire comprend des acides aminés protégés contre la dégradation ruminale, lesdits acides aminés protégés contre la dégradation ruminale étant éventuellement des acides aminés essentiels, ou
(ii) le complément alimentaire comprenant une source de protéines, de peptides, de peptides protégés contre la dégradation ruminale ou une combinaison de ceux-ci, ou
(iii) le régime alimentaire ajusté comprenant une source de protéines supplémentaire, une source de protéines différente, une quantité ajustée d'une source de protéines, une quantité réduite de fourrage grossier ou une combinaison de ceux-ci, par rapport au régime alimentaire initial.

3. Procédé selon la revendication 1 ou 2, comprenant en outre :
(i) la détermination d'une quantité d'énergie utilisable fournie par la quantité du régime alimentaire initial consommé par le ruminant, la détermination de la quantité d'énergie utilisable comprenant de préférence en outre la détermination de la teneur en protéines, en amidon et en fibres du régime alimentaire initial, ou
(ii) la détermination du besoin en énergie utilisable du ruminant, ou
(iii) la détermination du besoin en acides aminés du ruminant en fonction au moins en partie du besoin en énergie du ruminant et de la quantité d'énergie utilisable fournie par la quantité du régime initial consommé par le ruminant, ou
(iv) la détermination de l'efficacité microbienne ruminale, ladite détermination de l'efficacité microbienne ruminale reposant de préférence au moins en partie sur le taux de dilution, ou
(v) la détermination (a) d'un besoin en azote peptidique ou en azote d'acides aminés des microbes du rumen et (b) d'un besoin en azote ammoniacal des microbes du rumen, ou
(vi) toute combinaison des points (i), (ii), (iii), (iv) et (v).

4. Procédé selon la revendication 3, comprenant en outre :
la comparaison de l'azote peptidique ou de l'azote aminé fourni par le régime alimentaire initial au besoin en azote peptidique ou en azote aminé microbien afin d'obtenir une comparaison de l'azote peptidique ou de l'azote aminé, et
la comparaison de l'azote ammoniacal fourni par le régime alimentaire initial au besoin en azote ammoniacal des microbes afin d'obtenir une comparaison de l'azote ammoniacal,
la détermination de la quantité d'urée, de source de protéines, de peptides, d'acides aminés protégés contre la dégradation ruminale ou de toute combinaison de ceux-ci reposant de préférence en outre au moins en partie sur la comparaison de l'azote peptidique ou de l'azote aminé et sur la comparaison de l'azote ammoniacal.

5. Procédé selon l'une quelconque des revendications 2 à 4, dans lequel :
l'énergie utilisable est une énergie effective, et le régime alimentaire ajusté, le complément alimentaire ou la combinaison de ceux-ci fournit les grammes d'acides aminés essentiels par mégajoule d'énergie effective dans les plages suivantes : Lys 0,2-2, Met 0,05-1, His 0,1-1, Phe > 0,2, Thr > 0,2, Leu > 0,4, Val > 0,2, Arg 0,2-2, Ile > 0,2, Trp > 0,03, ou toute combinaison de celles-ci pour les animaux en croissance, de préférence dans les plages suivantes : Lys 0,4-0,8, Met 0,12-0,25, His 0,15-0,35, Phe ≥ 0,2, Thr ≥ 0,25, Leu ≥ 0,45, Val ≥ 0,25, Arg 0,4-0,8, Ile ≥ 0,2, Trp ≥ 0,03, ou toute combinaison de celles-ci, ou
l'énergie utilisable étant une énergie métabolisable, et le régime alimentaire ajusté, le complément alimentaire ou la combinaison de ceux-ci fournit les grammes d'acides aminés essentiels par mégajoule d'énergie métabolisable dans les plages suivantes : Lys 0,2-2,5, Met 0,1-0,6, His 0,1-1, Phe ≥ 1,5, Thr ≥ 0,2, Leu ≥ 0,7, Arg ≥ 0,3, Ile ≥ 0,3, Trp ≥ 0,07, ou toute combinaison de celles-ci pour les animaux en lactation, de préférence dans les plages suivantes : Lys 0,7-1, Met 0,2-0,3, His 0,25-0,35, Phe ≥ 0,4, Thr ≥ 0,35, Leu ≥ 0,84, Val ≥ 0,47, Arg ≥ 0,43, Ile ≥ 0,38, Trp ≥ 0,09, ou toute combinaison de celles-ci.

6. Système comprenant :
un ou plusieurs processeurs, et
un support de stockage lisible par ordinateur, sur lequel sont stockées des instructions exécutables par ordinateur qui, lorsqu'elles sont exécutées par le ou les processeurs, amènent le ou les processeurs à mettre en œuvre collectivement le procédé selon la revendication 1.

7. Système selon la revendication 6, dans lequel le support de stockage lisible par ordinateur contient des instructions exécutables par ordinateur supplémentaires qui, lorsqu'elles sont exécutées par le ou les processeurs, amènent le ou les processeurs à mettre en œuvre collectivement le procédé selon la revendication 3.

8. Système selon la revendication 7, dans lequel le support de stockage lisible par ordinateur contient des instructions exécutables par ordinateur supplémentaires qui, lorsqu'elles sont exécutées par le ou les processeurs :
(i) amènent le ou les processeurs à déterminer collectivement la prédiction du flux d'acides aminés alimentaires et d'acides aminés microbiens vers l'intestin grêle du ruminant en fonction au moins en partie de l'efficacité microbienne ruminale, d'une quantité d'énergie utilisable fournie par une quantité du régime alimentaire initial consommé par le ruminant, et de la teneur en protéines, en amidon et en fibres du régime alimentaire initial, ou
(ii) amènent le ou les processeurs à mettre en œuvre le procédé selon la revendication 4, ou
(iii) les points (i) et (ii).

9. Système selon l'une quelconque des revendications 6 à 8, dans lequel les supports de stockage lisibles par ordinateur contiennent collectivement des instructions exécutables par ordinateur supplémentaires qui, lorsqu'elles sont exécutées par le ou les processeurs :
(i) amènent le ou les processeurs à effectuer collectivement d'autres opérations comprenant :
(c1) la réception d'un ou plusieurs premiers signaux d'un système de formulation d'un régime alimentaire initial, le ou les premiers signaux transmettant des données concernant une formulation d'un régime alimentaire initial pour le ruminant, ou
(c2) la réception d'un ou plusieurs deuxièmes signaux transmettant des données concernant le ruminant, ou
(c3) la réception d'un ou plusieurs troisièmes signaux transmettant des données concernant un produit alimentaire disponible, un complément alimentaire disponible ou une combinaison de ceux-ci, ou
(c4) toute combinaison de (c1), (c2) et (c3) ; ou
(ii) amènent le ou les processeurs à sélectionner collectivement un ou plusieurs produits alimentaires, un ou plusieurs compléments alimentaires, ou toute combinaison de ceux-ci qui, en combinaison avec le régime alimentaire initial pour ruminant, satisfont la quantité déterminée d'urée, de source de protéines, de peptides, de peptides protégés contre la dégradation ruminale, d'acides aminés protégés contre la dégradation ruminale, ou toute combinaison de ceux-ci, ou
(iii) les points (i) et (ii).

10. Système selon la revendication 9, dans lequel :
le support de stockage lisible par ordinateur contient des instructions exécutables par ordinateur supplémentaires qui, lorsqu'elles sont exécutées par le ou les processeurs, amènent le ou les processeurs à générer collectivement un ou plusieurs quatrièmes signaux en fonction au moins en partie du ou des produits alimentaires sélectionnés, d'un ou de plusieurs compléments alimentaires, ou d'une combinaison de ceux-ci ; et
le ou les quatrièmes signaux amènent :
(d1) une interface utilisateur à afficher le ou les produits alimentaires sélectionnés, le ou les compléments alimentaires, ou des combinaisons de ceux-ci, ou
(d2) un distributeur alimentaire à ajuster automatiquement un régime alimentaire administré au ruminant afin d'inclure le ou les produits alimentaires sélectionnés, le ou les compléments alimentaires, ou une combinaison de ceux-ci, ou
(d3) les points (d1) et (d2).

11. Système selon l'une quelconque des revendications 6 à 10, comprenant en outre une interface d'entrée/sortie située de manière à :
(e1) communiquer électroniquement avec un système de formulation de régime alimentaire initial qui détermine le régime alimentaire initial pour ruminant, ou
(e2) communiquer électroniquement avec une mémoire ou une base de données dans lesquelles sont stockées des données concernant les produits alimentaires et les compléments alimentaires disponibles pour le ruminant, ou
(e3) communiquer électroniquement avec une interface utilisateur comprenant un dispositif d'entrée/sortie, un écran ou les deux, ou
(e4) communiquer électroniquement avec un système d'alimentation qui distribue des aliments au ruminant et/ou le commander automatiquement, ou
(e5) toute combinaison de (e1), (e2), (e3) et (e4).

12. Système selon l'une quelconque des revendications 6 à 11, dans lequel le support de stockage lisible par ordinateur contient des instructions exécutables par ordinateur supplémentaires qui, lorsqu'elles sont exécutées par le ou les processeurs, amènent le ou les processeurs à générer collectivement un ou plusieurs signaux de commande relatifs à un système d'alimentation qui amènent le système d'alimentation à préparer un régime alimentaire ajusté, un complément alimentaire ou une combinaison de ceux-ci, comprenant la quantité déterminée d'urée, de source de protéines, de peptides, de peptides protégés contre la dégradation ruminale, d'acides aminés protégés contre la dégradation ruminale ou une combinaison de ceux-ci.

13. Support(s) non transitoire(s) lisible(s) par ordinateur sur lequel/lesquels sont stockées collectivement des instructions exécutables par ordinateur qui, lorsqu'elles sont exécutées par un ou plusieurs dispositifs informatiques, configurent le ou les dispositifs informatiques à exécuter au moins une partie du procédé selon la revendication 1.

14. Support(s) non transitoire(s) lisible(s) par ordinateur selon la revendication 13, les instructions stockées sur le ou les supports non transitoires lisibles par ordinateur comprenant :
(a1) des instructions qui amènent le ou les dispositifs informatiques à recevoir un ou plusieurs premiers signaux transmettant des données concernant une formulation alimentaire initiale pour le ruminant, ou
(a2) des instructions qui amènent le ou les dispositifs informatiques à recevoir un ou plusieurs deuxièmes signaux transmettant des données concernant le ruminant, ou
(a3) des instructions qui amènent le ou les dispositifs informatiques à recevoir un ou plusieurs troisièmes signaux transmettant des données concernant un produit alimentaire disponible, un complément alimentaire disponible ou une combinaison de ceux-ci, ou
(a4) toute combinaison de (a1), (a2) et (a3).

15. Support(s) non transitoire(s) lisible(s) par ordinateur selon la revendication 13 ou 14, les instructions stockées sur le ou les supports non transitoires lisibles par ordinateur comprenant des instructions qui amènent le ou les dispositifs informatiques à sélectionner un ou plusieurs produits alimentaires, un ou plusieurs compléments alimentaires, ou toute combinaison de ceux-ci, en fonction au moins en partie, de la quantité déterminée d'urée, de source de protéines, de peptides, de peptides protégés contre la dégradation ruminale, d'acides aminés protégés contre la dégradation ruminale, ou de toute combinaison de ceux-ci.

16. Support(s) non transitoire(s) lisible(s) par ordinateur selon l'une quelconque des revendications 13 à 15,
les instructions stockées sur le ou les supports non transitoires lisibles par ordinateur comprennent des instructions qui amènent le ou les dispositifs informatiques à transmettre un ou plusieurs quatrièmes signaux ;et
le ou les quatrièmes signaux amenant :
(b1) une interface utilisateur à afficher la quantité déterminée d'urée, de source de protéines, de peptides, de peptides protégés contre la dégradation ruminale, d'acides aminés protégés contre la dégradation ruminale ou toute combinaison de ceux-ci, ou
(b2) une interface utilisateur à afficher le ou les produits alimentaires sélectionnés, les ou les compléments alimentaires, ou toute combinaison de ceux-ci, ou
(b3) un distributeur alimentaire à ajuster un régime alimentaire administré au ruminant afin d'inclure le ou les produits alimentaires sélectionnés, le ou les compléments alimentaires, ou une combinaison de ceux-ci, ou
(b4) toute combinaison de (b1), (b2), (b3) et (b4).
